# EUROPÄISCHE PATENTANMELDUNG

(11) **EP 2 886 533 A1**
(43) Veröffentlichungstag der Anmeldung: **24.06.2015**
(21) Anmeldenummer: 14199205.7
(22) Anmeldetag: 26.08.2010
(51) Int. Cl.: C07D 213/30, C07D 333/16, C07F 9/145, C07F 9/6571, C07F 9/6574, C07F 9/6584, C07F 15/04, C07B 41/06, C07B 43/04, C07B 43/08, C07B 51/00, C07B 53/00, C07C 39/14, C07C 39/15, C07C 43/205, C07C 43/295, C07C 67/303, C07C 37/20, C07F 9/655, C07C 209/26, C07C 45/50

(54) **Verwendung von Organophosphorverbindungen basierend auf Tetraphenol(TP)-substituierten Strukturen**

(30) Priorität: 31.08.2009 DE 102009029050
(62) Teilanmeldung aus: 10745266.6
(71) Anmelder: Evonik Degussa GmbH, 45128 Essen (DE)
(72) Erfinder: Christiansen, Andrea, 18119 Rostock (DE); Franke, Robert, 45772 Marl (DE); Fridag, Dirk, 45721 Haltern am See (DE); Hess, Dieter, 45770 Marl (DE); Kreidler, Burkard, 45219 Essen (DE); Vogt, Dieter, Edinburgh, EH10 5UN (GB); Bini, Laura, 6211 EB Maastricht (NL); Janssen, Michèle, 5645 LB Eindhoven (NL); Hamers, Bart, 5961 VG Horst (NL)

(57) **Zusammenfassung**

Die Erfindung betrifft ein Verfahren zur Hydrocyanierung von Pentennitril oder Butadien-haltigen Strömen unter Verwendung einer katalytisch wirksamen Zusammensetzung, enthaltend ein Übergangsmetall der Gruppen 8 bis 10, dadurch gekennzeichnet, dass die katalytisch wirksame Zusammensetzung eine organische Phosphorverbindung der Formel 2 aufweist, 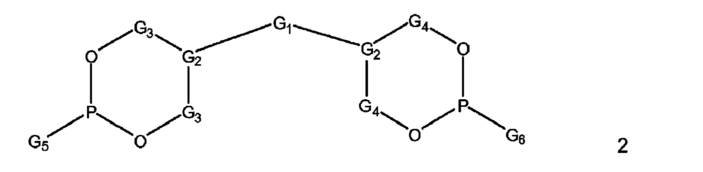 worin:
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist;
G3 und G4 gleich oder verschieden sind und eine mindestens einfach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden sind;
G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Acyl, N-Alkyl, N-Aryl, N-Heteroaryl, N-Cycloalkyl, N-Silyl.

## Beschreibung

Die vorliegende Erfindung betrifft neue organische Verbindungen, ihre Phosphorderivate bzw. organische Phosphorverbindungen sowie deren Komplexverbindungen mit Übergangsmetallen, Verfahren zu deren Herstellung und Verwendung in katalytischen Reaktionen.

Die Reaktionen zwischen Olefinverbindungen, Kohlenmonoxid und Wasserstoff in Gegenwart eines Katalysators zu den um ein C-Atom reicheren Aldehyden ist als Hydroformylierung (Oxierung) bekannt. Als Katalysatoren in diesen Reaktionen werden häufig Verbindungen der Übergangsmetalle der 8. bis 10. Gruppe des Periodensystems der Elemente verwendet, insbesondere Verbindungen des Rhodiums und des Kobalts. Die Hydroformylierung mit Rhodiumverbindungen bietet im Vergleich zur Katalyse mit Kobaltverbindungen in der Regel den Vorteil höherer Selektivität und ist damit meistens wirtschaftlicher. Bei der durch Rhodium katalysierten Hydroformylierung werden zumeist Komplexe eingesetzt, die aus Rhodium und bevorzugt aus trivalenten Phosphorverbindungen als Liganden bestehen. Bekannte Liganden sind beispielsweise Verbindungen aus den Klassen der Phosphine, Phosphite und Phosphonite. Eine Übersicht über Hydroformylierung von Olefinen findet sich in B. CORNILS, W. A. HERRMANN, "Applied Homogeneous Catalysis with Organometallic Compounds", Vol. 1&2, VCH, Weinheim, New York, 1996.

Jedes Katalysatorsystem (Kobalt oder Rhodium) hat seine spezifischen Vorzüge. Je nach Einsatzstoff und Zielprodukt werden unterschiedliche Katalysatorsysteme verwendet. Arbeitet man mit Rhodium und Triphenylphosphin, lassen sich α-Olefine bei niedrigeren Drücken hydroformylieren. Als phosphorhaltiger Ligand wird in der Regel Triphenylphosphin im Überschuss verwendet, wobei ein hohes Ligand/Rhodium-Verhältnis erforderlich ist, um die Selektivität der Reaktion zum kommerziell erwünschten n-Aldehydprodukt zu erhöhen.

US-A-4,694,109 und US-A-4,879,416 betreffen Bisphosphinliganden und ihren Einsatz in der Hydroformylierung von Olefinen bei niedrigen Synthesegasdrücken. Besonders bei der Hydroformylierung von Propen werden mit Liganden dieses Typs hohe Aktivitäten und hohe n/i-Selektivitäten erreicht.

In WO-A-95/30680 werden zweizähnige Phosphinliganden und ihr Einsatz in der Katalyse, unter anderem auch in Hydroformylierungsreaktionen, beschrieben.

Ferrocenverbrückte Bisphosphine werden beispielsweise in US-A-4,169,861, US-A-4,201,714 und US-A-4,193,943 als Liganden für Hydroformylierungen offenbart.

Der Nachteil von zweizähnigen Phosphinliganden ist die relativ aufwendige Herstellung. Daher ist es oftmals nicht rentabel, solche Systeme in technischen Prozessen einzusetzen.

Rhodium-Monophosphit-Komplexe sind geeignete Katalysatoren für die Hydroformylierung von verzweigten Olefinen mit innenständigen Doppelbindungen, jedoch ist die Selektivität für endständig hydroformylierte Verbindungen gering.

Aus EP-A-0 155 508 ist die Verwendung von bisarylensubstituierten Monophosphiten bei der rhodiumkatalysierten Hydroformylierung von sterisch gehinderten Olefinen, z. B. Isobuten bekannt.

Rhodium-Bisphosphit-Komplexe katalysieren die Hydroformylierung von linearen Olefinen mit end- und innenständigen Doppelbindungen, wobei überwiegend endständig hydroformylierte Produkte entstehen, dagegen werden verzweigte Olefine mit innenständigen Doppelbindungen nur in geringem Maße umgesetzt. Diese Phosphite ergeben bei ihrer Koordination an ein Übergangsmetallzentrum Katalysatoren von gesteigerter Aktivität, doch ist das Standzeitverhalten dieser Katalysatorsysteme, unter anderem wegen der Hydrolyseempfindlichkeit der Phosphitliganden, unbefriedigend. Durch den Einsatz von substituierten Bisaryldiolen als Edukte für die Phosphitliganden, wie in EP-A-0 214 622 oder EP-A-0 472 071 beschrieben, konnten erhebliche Verbesserungen erreicht werden.

Der Literatur zufolge sind die Rhodiumkomplexe dieser Liganden äußerst aktive Hydroformylierungskatalysatoren für α-Olefine. In US-A-4,668,651, US-A-4,748,261 und US-A-4,885,401 werden Polyphosphitliganden beschrieben, mit denen α-Olefine, aber auch 2-Buten mit hoher Selektivität zu den terminal hydroformylierten Produkten umgesetzt werden können. In US-A-5,312,996 werden zweizähnige Liganden dieses Typs auch zur Hydroformylierung von Butadien eingesetzt.

Aus dem Bereich der lichtempfindlichen, filmbildenden Materialien - Photolacke oder auch im Englischen Photoresists genannt - ist eine Substanzklasse bekannt, die unter dem Begriff Tetraphenole zusammengefasst wird. In JP 05034915 und JP 2004277358 werden z. B. solche Vertreter beschrieben. Journal of the Chemical Society, Perkin Transactions 1: Organic and Bio-Organic Chemistry (1972-1999) (1994), (13), 1879-82 und JP 2004277358 zeigen Wege zur Herstellung von den benötigten Vorstufen sowie zu den Tetraphenol-artigen Verbindungen auf.

In Macromolecules (Washington, DC, United States) (2008), 41(20), 7306-7315 wird z. B. die Verwendung von Tetraphenol-artigen Verbindungen bei der Ring-Öffnungspolymerisation von Ethylenoxid und Propylenoxid beschrieben.

Obgleich die zuvor genannten Bisphosphite gute Komplexliganden für Rhodium-basierte Hydroformylierungskatalysatorsysteme sind, ist es wünschenswert, neuartige leicht herstellbare Phosphite zur weiteren Verbesserung ihrer Wirksamkeit beispielsweise in der Hydroformylierung zu entwickeln.

So ist es eine Aufgabe der vorliegenden Erfindung organische Verbindungen, die in ihrer Struktur den Tetraphenolen zugeordnet werden können, herzustellen und in entsprechende Phosphorderivate umzusetzen.

Dabei ist es mit eine Aufgabe, dass die Herstellung der organischen Verbindungen wie auch der daraus abgeleiteten organischen Phosphorverbindungen technisch und ökonomisch unaufwendig erfolgt. Dabei steht ein modularer Aufbau der organischen Phosphorverbindungen im Vordergrund der Aufgabe, um einerseits variable Strukturen in wenigen Herstellungsschritten zu generieren sowie andererseits dazu auf großtechnisch und günstig verfügbare Ausgangsstoffe bzw. Edukte zuzugreifen.

Diese Phosphorderivate bzw. die eingangs genannten organischen Phosphorverbindungen werden mit Übergangsmetallen zu katalytisch wirksamen Zusammensetzungen weiter verarbeitet. Weiterhin ist es eine Aufgabe diese katalytisch wirksamen Zusammensetzungen anzuwenden in Reaktionen von kleinen Molekülen, wie z.B. CO, HCN, Wasserstoff oder auch Aminen, in reiner Form wie auch in Gemischen, mit ungesättigten Kohlenwasserstoffverbindungen. Mit ist es eine Aufgabe, dass diese katalytisch wirksamen Zusammensetzungen, z. B. in der Hydroformylierung von ungesättigten Kohlenwasserstoffverbindungen, eine hohe Standzeit und damit verbunden die als Liganden verwendeten organischen Phosphorverbindungen eine geringe Hydrolyseempfindlichkeit sowie eine hohe Selektivität zum linearen, d. h. n-hydroformylierten Produkt, aufweisen.

Gegenstand der Erfindung sind organische Verbindungen der Formel 1 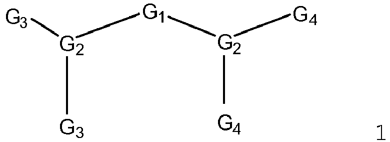 worin:
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 und G4 gleich oder verschieden, mit jeweils einer OH-Gruppe substituiert sind und eine mindestens zweifach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 verbunden sind.

In einer bevorzugten Ausführungsform reduziert sich die Vielfalt der möglichen Strukturen, die aus der Formel 1 abgeleitet werden können, dadurch, dass folgende Einschränkungen eingeführt werden, wobei:
G1 ein mindestens disubstituierter 1,2-, 1,3- oder 1,4- Phenylrest ist;
G2 ein C1-Alkylrest ist, der tertiär oder quartär substituiert ist;
G3 und G4 gleich sind und einen mindestens disubstituierten aromatischen Rest mit jeweils einer OH-Gruppe darstellen, ausgewählt aus der Gruppe der Aromaten, der Heteroaromaten, der kondensierten Aromatensysteme oder der kondensierten heteroaromatischen Systeme mit beliebiger weiterer Substitution.

Beispielhaft für diese bevorzugte Ausführungsform sind folgende Strukturen, wobei G1 eine 1,3-Substituierung aufweist:

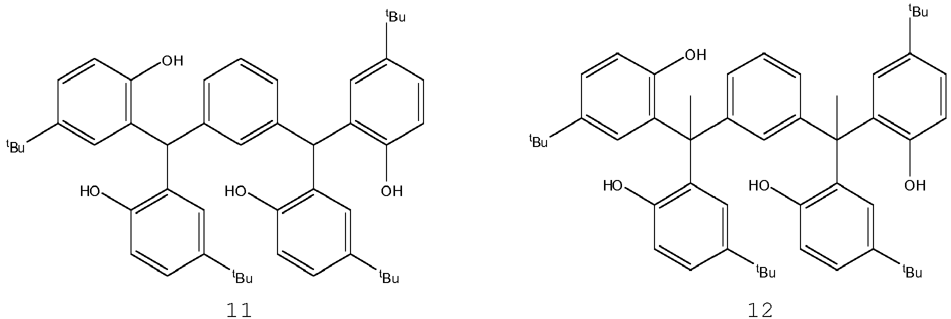

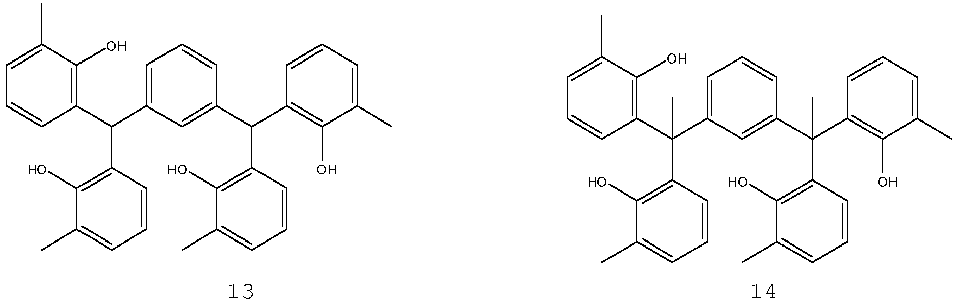

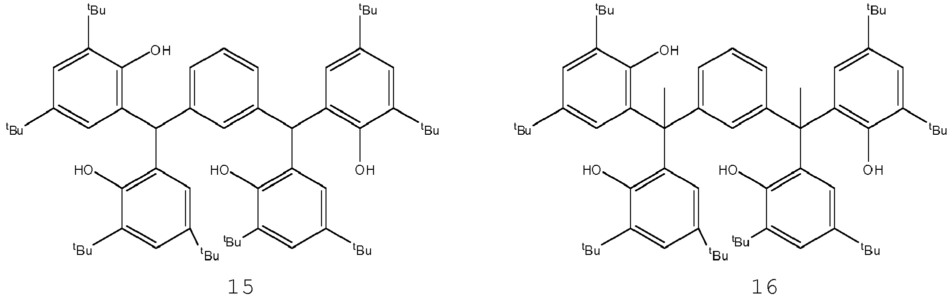

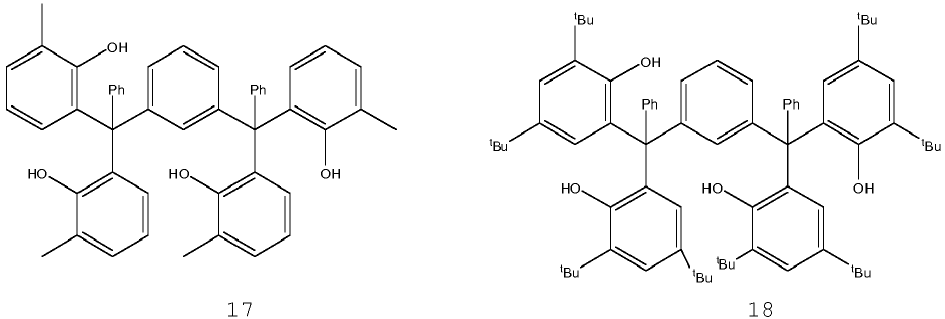

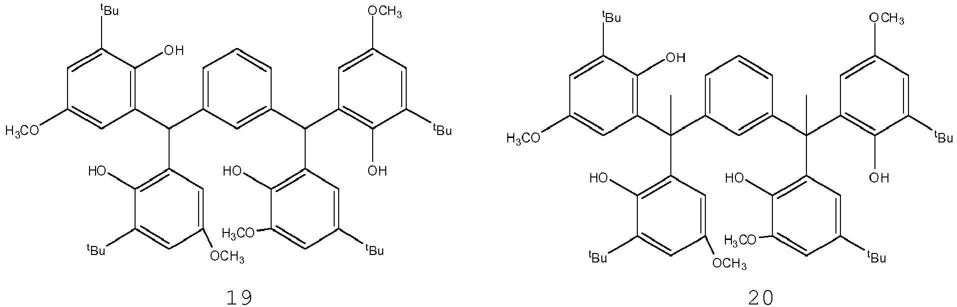

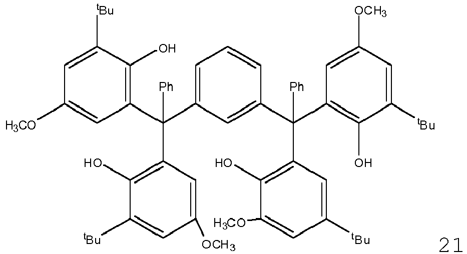

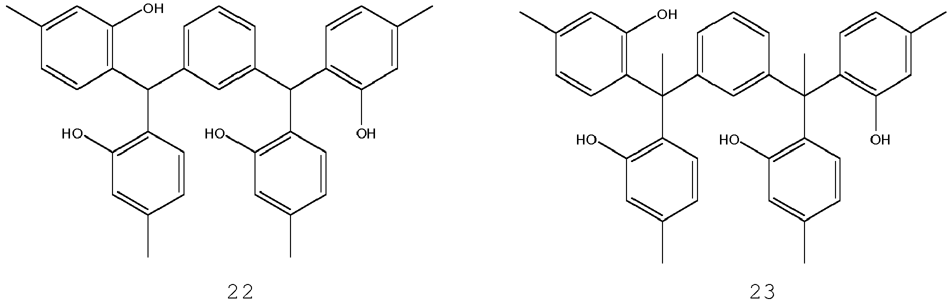

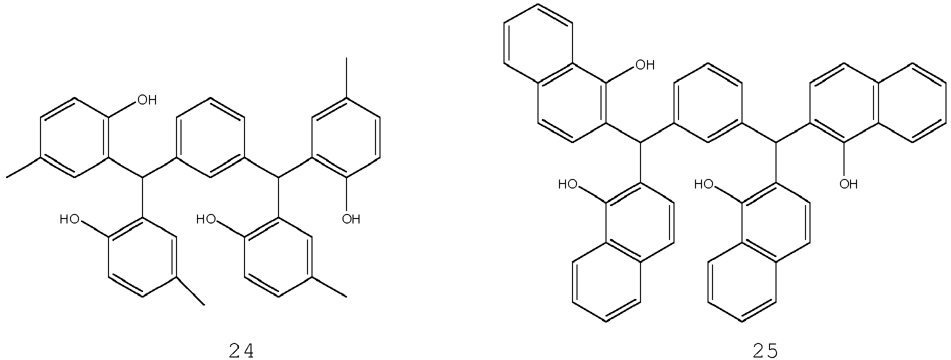

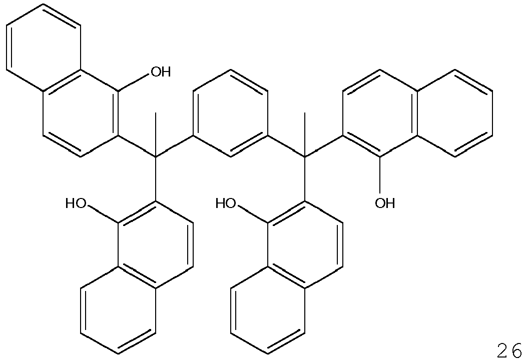

Beispielhaft für diese bevorzugte Ausführungsform ist z. B. auch: 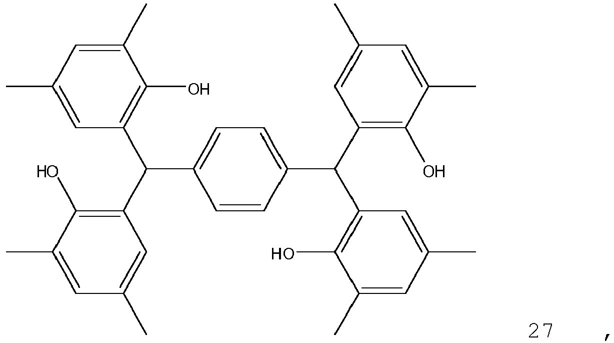 wobei G1 eine 1 4-Substitution aufweist, während die nachfolgende Ausführung der Formel 28 beispielhaft einen Vertreter darstellt, worin G1 eine 1,2-Substitution zeigt:

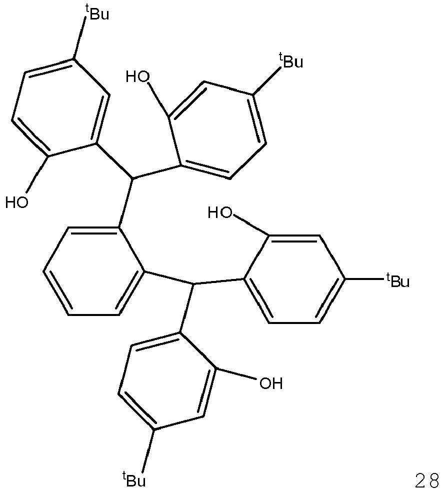

Beispielhafte Vertreter dieser Ausführungsform sind auch folgende Derivate der Formeln 29 bis 38, welche für G1 eine Trisubstitution, einen Heteroaromaten oder ein kondensiertes aromatisches System aufweisen:

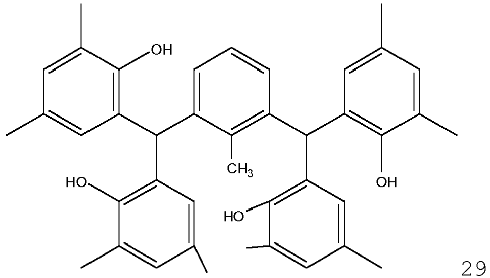

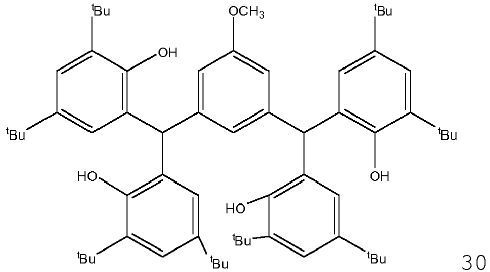

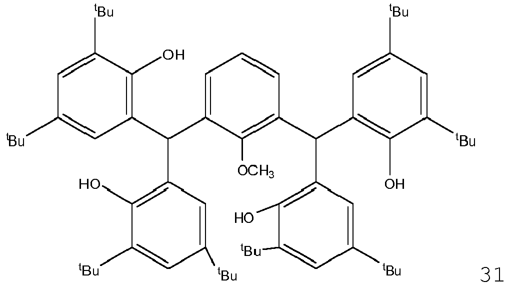

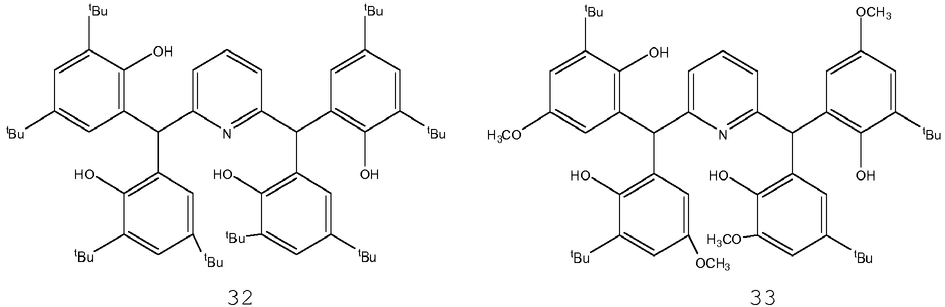

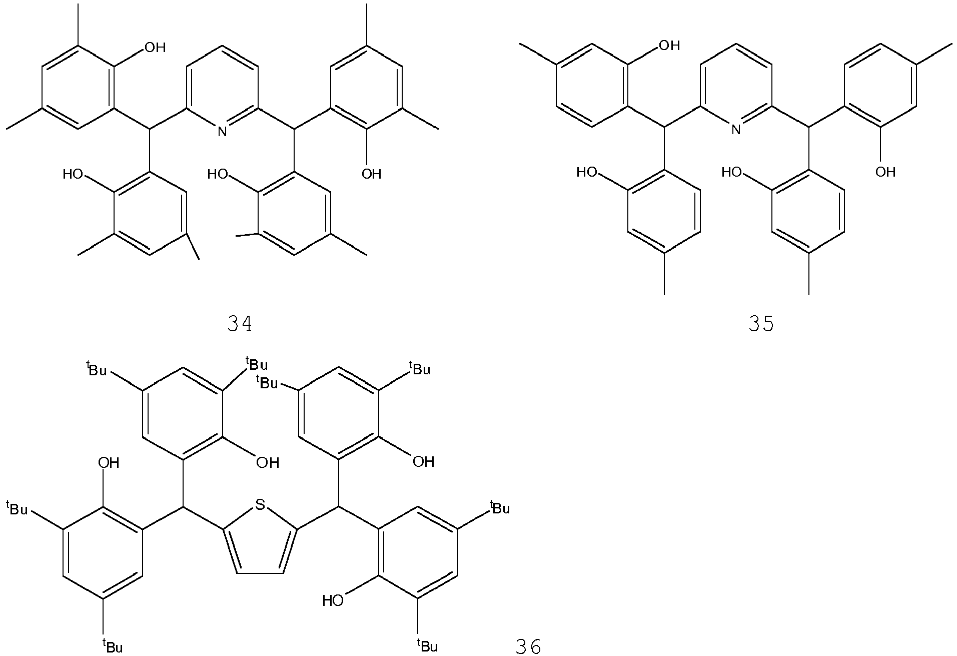

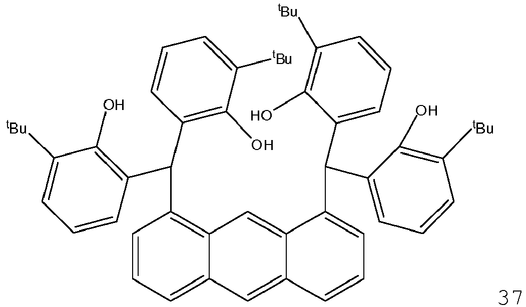

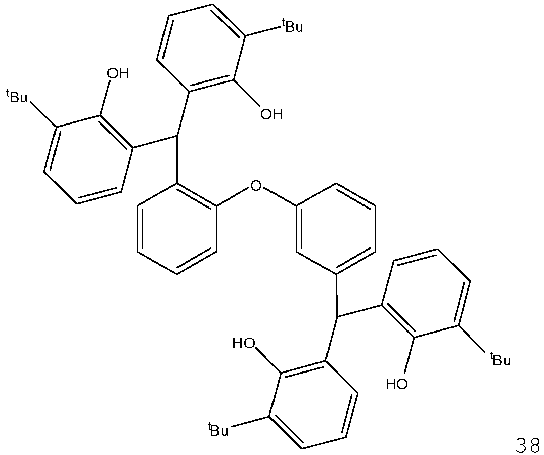

In einer weiteren, bevorzugten Ausführungsform gemäß der Formel 1 ist bzw. sind:
G1 ein mindestens disubstituierter 1,3- Phenylrest;
G2 ein C1-Alkylrest, der mit Wasserstoff, Methyl, Ethyl, Isopropyl, Isobutyl, Trifluormethyl, oder Aryl substituiert ist;
G3 und G4 gleich und ein mindestens disubstituierter mit OH, C1 bis C6 O-Alkyl sowie C1 bis C6-Alkyl versehener aromatischer Rest sind.

Beispielhafte Vertreter dieser Ausführungsform sind z.B. in den Formeln 11 bis 26 sowie 29 bis 31 wiedergegeben. Die Herstellung erfolgt in der Art und Weise, wie sie in den erfindungsgemäßen Herstellungsbeispielen nachfolgend offenbart wird.

In einer anderen, bevorzugten Ausführungsform gemäß der Formel 1 ist bzw. sind:
G1 ein 1,3-disubstituierter Phenylrest;
G2 ein C1-Alkylrest, der mit Wasserstoff, Methyl, Ethyl, Isopropyl, Isobutyl, Trifluormethyl, oder Aryl substituiert ist;
G3 und G4 gleich und ein mit OH sowie 2 tert.-Butyl-Gruppen trisubstituierter aromatischer Rest.

Beanspruchte Vertreter dieser Ausführungsform sind z.B. in den Formeln 12, 15, 16 und 18 offenbart und können auf Basis der erfindungsgemäßen Herstellungsbeispiele synthetisiert werden. In den erfindungsgemäßen Herstellungsbeispielen werden die beanspruchten Vertreter mit TP0, TP'0 und TP"0 der Verständlichkeit halber bezeichnet.

Weiter sind Gegenstand der Erfindung organische Phosphorverbindungen der Formel 2: 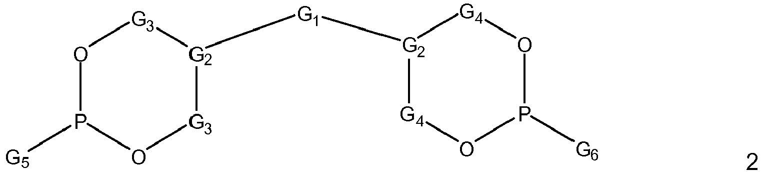 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 und G4 gleich oder verschieden sind und eine mindestens einfach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden sind; G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

In einer besonderen Ausführungsform der Formel 2 ist bzw. sind:
G1 ein mindestens disubstituierter 1,2-, 1,3- oder 1,4- Phenylrest;
G2 ein C1-Alkylrest, der tertiär oder quartär substituiert ist;
G3 und G4 gleich und stellen einen mindestens einfach substituierten aromatischen Rest dar, ausgewählt aus der Gruppe der Aromaten, der Heteroaromaten, der kondensierten Aromatensysteme oder der kondensierten heteroaromatischen Systeme mit beliebiger weiterer Substitution;
G5 und G6 gleich, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Alkyl, N-Aryl, N-Heteroaryl, N-Cycloalkyl, N-Silyl.

In einer besonders bevorzugten Ausführungsform der Formel 2 ist bzw. sind:
G1 ein 1,3-disubstituierter Phenylrest;
G2 ein C1-Alkylrest, der mit Wasserstoff oder Methyl substituiert ist;
G3 und G4 gleich, ausgewählt aus der Gruppe tert.-Butylphenoxy, Methoxy-tert.-Butylphenoxy, oder di-tert.-Butylphenoxy;
G5 und G6 gleich, ausgewählt aus der Gruppe tert.-Butylphenoxy, Methoxy-tert.-Butylphenoxy, Naphthoxy, di-tert.-Butylphenoxy, Methyl-tert.-Butylphenoxy oder Pyrrol. Die nach diesen Ausführungsformen beanspruchten organischen Phosphorverbindungen - auch Tetraphenol-Liganden oder Tetraphenol substituierte Bisphosphite genannt - sind nachfolgend in den erfindungsgemäßen Herstellungsbeispielen weiter offenbart und charakterisiert.

Der Verständlichkeit halber werden die beanspruchten Tetraphenol-Liganden in den erfindungsgemäßen Herstellungsbeispielen mit TP1 bis TP7 bezeichnet. Die zu den beanspruchten Tetraphenol-Liganden gehörigen Strukturen sind ebenfalls in den erfindungsgemäßen Herstellungsbeispielen im Schema 1 offenbart.

Weitere Ausführungsformen der erfindungsgemäßen Formel 2 sind beispielhaft in der nachfolgenden Struktur, die mit TP bezeichnet wird, aufgeführt:

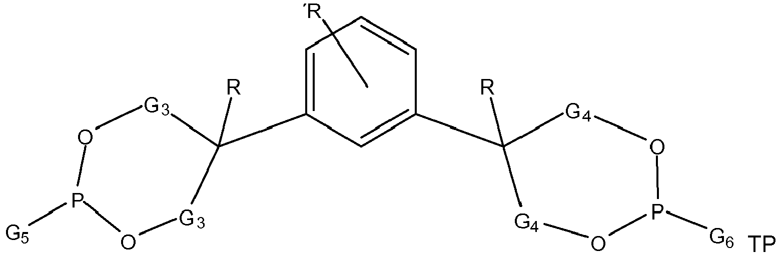

Wobei gemäß der Formel TP G3 und G4 gleich oder verschieden sind und eine mindestens einfach substituierte cyclische Struktur darstellen, z. B.

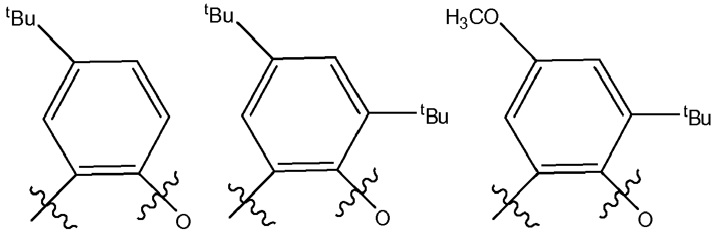

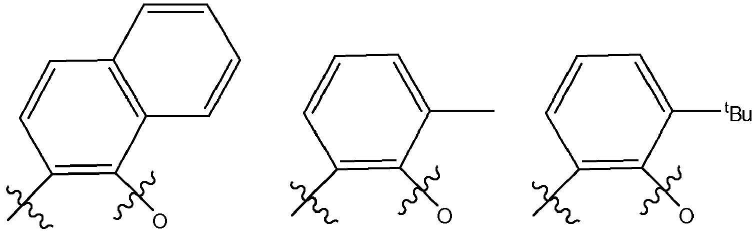

Wobei G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Acyl, N-Alkyl, N-Aryl, N-Heteroaryl, N-Cycloalkyl, N-Silyl; z. B. 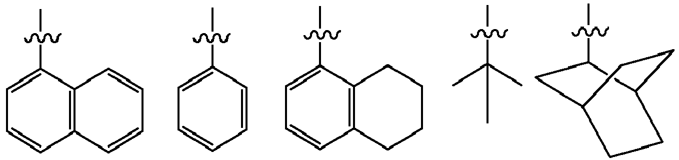 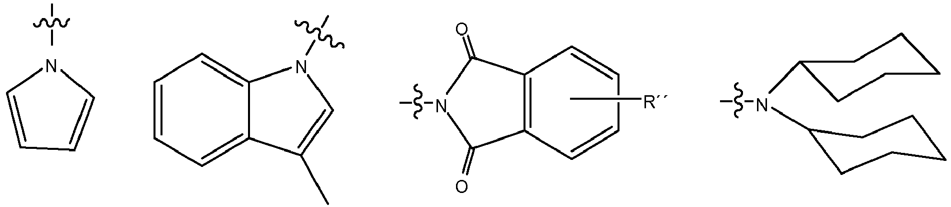 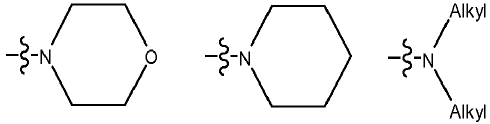 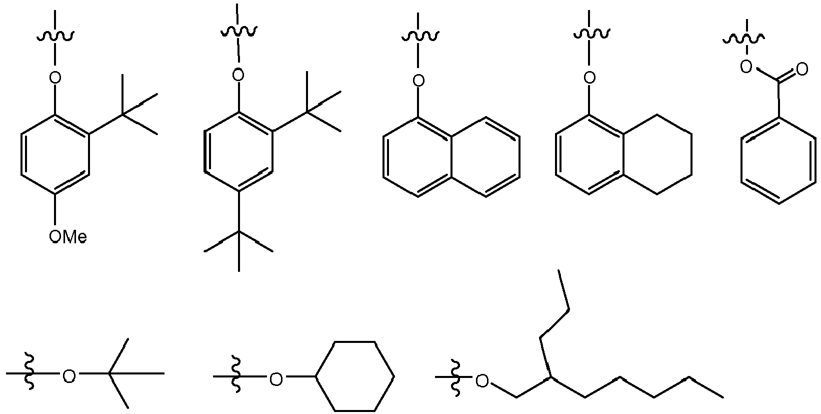 und R z. B. gleich Wasserstoff, Methyl, Trifluormethyl, oder Phenyl darstellt;

Sowie R' beispielhaft durch folgende Reste dargestellt wird:

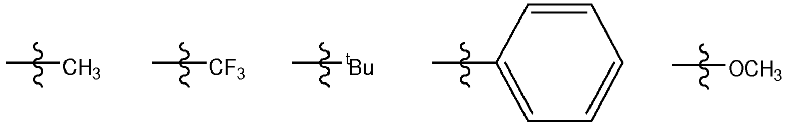

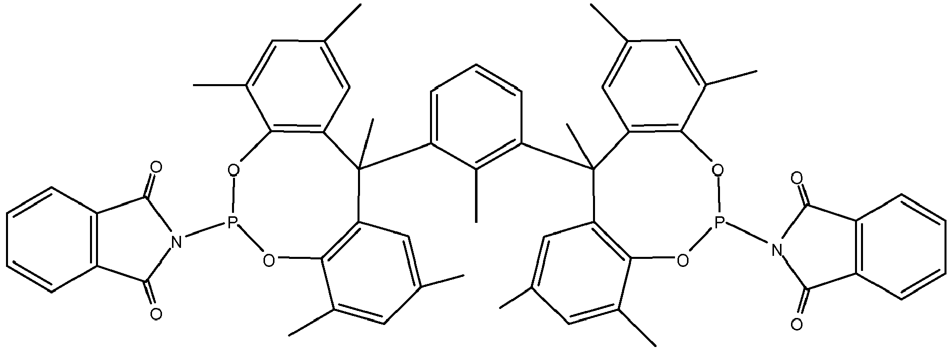

Auch sind Gegenstand der vorliegenden Erfindung organische Phosphorverbindungen der Formel 3 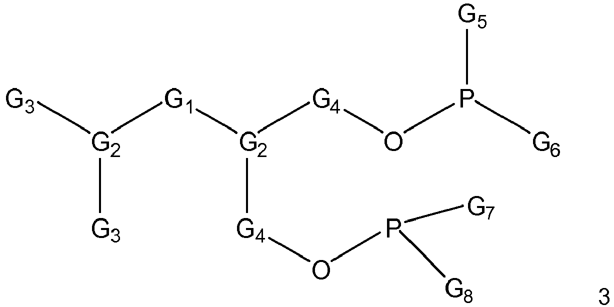 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 mit jeweils einer OH-Gruppe substituiert ist und eine mindestens zweifach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 verbunden ist;
G4 eine mindestens einfach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden ist;
G5, G6, G7 und G8 jeweils gleiche oder verschiedene monovalent mit P verbundene Einheiten oder G5 jeweils mit G6 und G7 jeweils mit G8 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Weiter sind Gegenstand der Erfindung organische Phosphorverbindungen der Formel 4 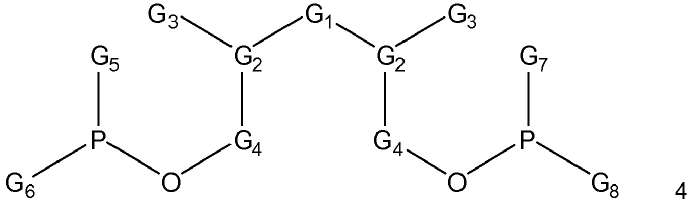 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 mit jeweils einer OH-Gruppe substituiert ist und eine mindestens zweifach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 verbunden ist;
G4 eine mindestens einfach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden ist;
G5, G6, G7 und G8 jeweils gleiche oder verschiedene monovalent mit P verbundene Einheiten oder G5 jeweils mit G6 und G7 jeweils mit G8 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Auch sind Gegenstand der vorliegenden Erfindung organische Phosphorverbindungen der Formel 5 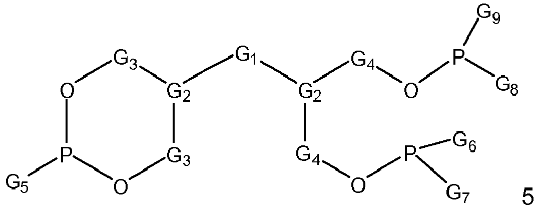 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten oder der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 und G4 gleich oder verschieden sind und eine mindestens zweifach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden sind;
G5, G6, G7, G8 und G9 jeweils gleiche oder verschiedene monovalent mit P verknüpfte Einheiten oder G6 jeweils mit G7 und G8 jeweils mit G9 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Weiter sind Gegenstand der Erfindung organische Phosphorverbindungen der Formel 6 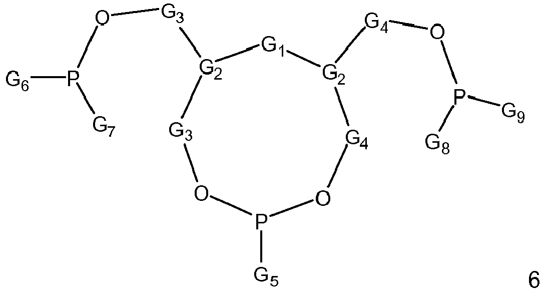 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 und G4 gleich oder verschieden sind und eine mindestens zweifach substituierte cyclische Struktur darstellen, ausgewählt sind aus der Gruppe der Aromaten oder der Heteroaromaten, der kondensierten Aromatensysteme oder der kondensierten heteroaromatischen Systeme mit beliebiger weiterer Substitution, welche jeweils monovalent mit G2 und O verbunden sind;
G5, G6, G7, G8 und G9 jeweils gleiche oder verschiedene monovalent mit P verknüpfte Einheiten oder G6 jeweils mit G7 und G8 jeweils mit G9 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Auch sind Gegenstand der vorliegenden Erfindung organische Phosphorverbindungen der Formel 7 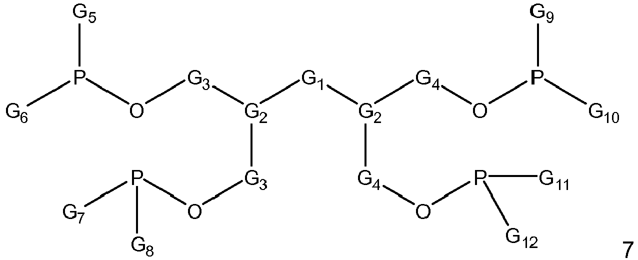 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 und G4 gleich oder verschieden sind und eine mindestens zweifach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden sind;
G5, G6, G7, G8, G9, G10, G11 und G12 jeweils gleiche oder verschiedene monovalent mit P verknüpfte Einheiten oder G5 jeweils mit G6, G7 jeweils mit G8, G9 jeweils mit G10 und G11 jeweils mit G12 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

In einer besonderen Ausführungsform gemäß der Formel 7 ist bzw. sind:
G1 ein 1,3-disubstituierter Phenylrest;
G2 ein C1-Alkylrest, der mit Wasserstoff, Methyl, Ethyl, Isopropyl, Isobutyl, Trifluormethyl oder Aryl substituiert ist;
G3 und G4 gleich, ausgewählt aus der Gruppe tert.-Butylphenoxy oder di-tert.-Butylphenoxy; G5, G6, G7, G8, G9, G10, G11 und G12 jeweils gleiche oder verschiedene monovalent mit P verknüpfte Einheiten oder G5 jeweils mit G6, G7 jeweils mit G8, G9 jeweils mit G10 und G11 jeweils mit G12 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Beispielhafte Strukturen dieser Ausführungsform sind:

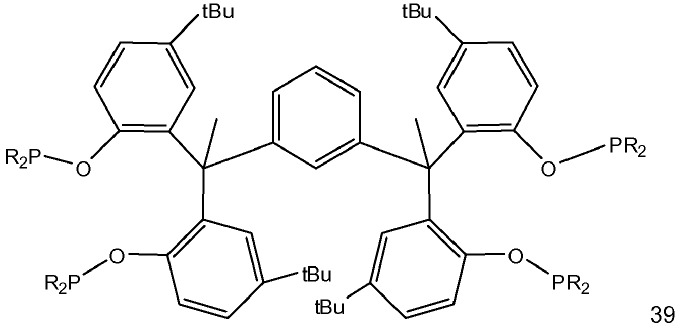

Wobei PR₂ in der Formel 39 z.B. folgende Substituierung aufweist:

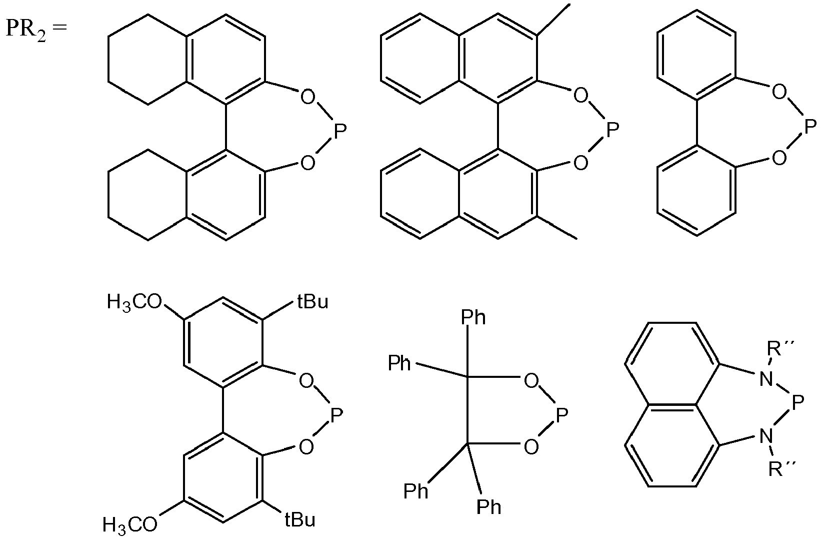

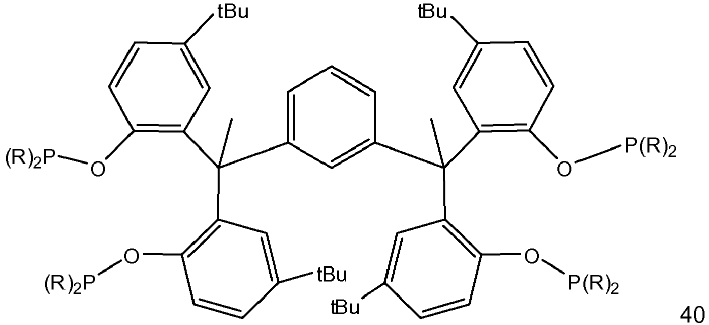

Wobei R in Formel 40 beispielhaft folgende Reste darstellt:

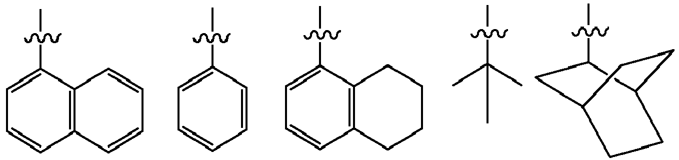

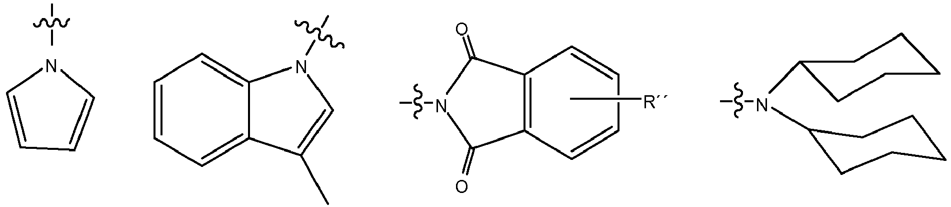

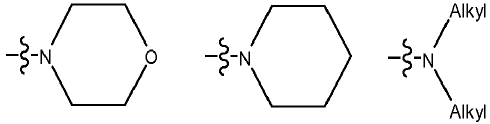

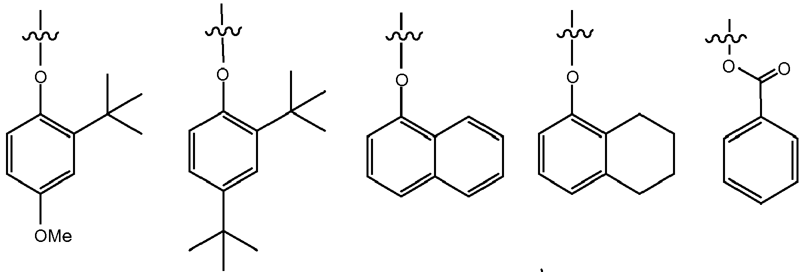

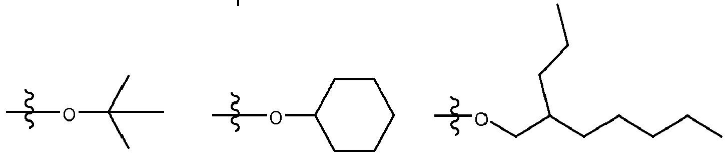

Der Vertreter der Formel 40 mit R = 2,4-di-tert.-Butylphenoxy ist eine erfindungsgemäß beanspruchte Ausführungsform und stellt ein Tetraphenol basiertes Tetraphosphit dar, welche unter der Benutzung der Formel TP8 weiter in den erfindungsgemäßen Herstellungsbeispielen offenbart wird.

Weiter sind Gegenstand der Erfindung organische Phosphorverbindungen der Formel 8 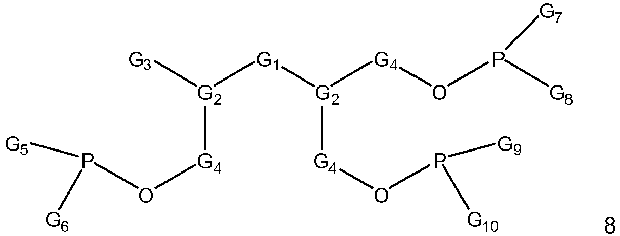 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 mit jeweils einer OH-Gruppe substituiert ist und eine mindestens zweifach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche monovalent mit G2 verbunden ist;
G4 eine mindestens einfach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden ist;
G5, G6, G7, G8, G9 und G10 jeweils gleiche oder verschiedene monovalent mit P verbundene Einheiten oder G5 jeweils mit G6, G7 jeweils mit G8 und G9 jeweils mit G10 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Auch sind Gegenstand der vorliegenden Erfindung organische Phosphorverbindungen der Formel 9 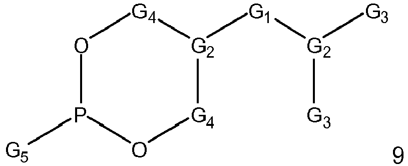 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist;
G3 mit jeweils einer OH-Gruppe substituiert ist und eine mindestens zweifach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche monovalent mit G2 verbunden ist;
G4 eine mindestens einfach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden ist;
G5 eine monovalent mit P verknüpfte Einheit darstellt, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Weiter sind Gegenstand der Erfindung organische Phosphorverbindungen der Formel 10 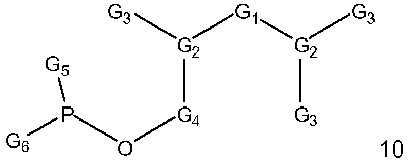 worin:
O jeweils einem Sauerstoffatom und
P jeweils einem Phosphoratom entsprechen;
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist; G3 mit jeweils einer OH-Gruppe substituiert ist und eine mindestens zweifach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche monovalent mit G2 verbunden ist;
G4 eine mindestens einfach substituierte cyclische Struktur darstellt, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden ist;
G5, und G6 jeweils gleiche oder verschiedene monovalent mit P verknüpfte Einheiten oder G5 jeweils mit G6 kovalent verknüpfte und monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralkyl, N-Acyl, N-Alkyl, N-Aryl, N-Heteroaryl, N-Cycloalkyl, N-Silyl.

Auch Gegenstand der Erfindung sind Metallkomplexe, enthaltend ein Metall der 4., 5., 6., 7., 8., 9. oder 10. Gruppe des Periodensystems der Elemente und ein oder mehrere organische Phosphorverbindungen, die in den erfindungsgemäßen Herstellungsbeispielen mit TP1, TP2, TP3, TP4 oder TP5 aufgeführt sind.

In einer besonderen Ausführungsform sind die erfindungsgemäßen Metallkomplexe dadurch gekennzeichnet, dass das Metall Rhodium, Palladium, Nickel, Platin, Kobalt oder Ruthenium ist.

In einer weiteren Ausführungsform finden mindestens eine der organischen Phosphorverbindungen, die in den erfindungsgemäßen Herstellungsbeispielen mit TP1, TP2, TP3, TP4 oder TP5 bezeichnet sind und/oder ein Metallkomplex, basierend auf Rhodium, Palladium, Nickel, Platin, Kobalt oder Ruthenium, Verwendung in der Katalyse.

In einer weiteren Ausführungsform finden mindestens eine der organischen Phosphorverbindungen, die in den erfindungsgemäßen Herstellungsbeispielen mit TP1, TP2, TP3, TP4 oder TP5 bezeichnet sind und/oder ein Metallkomplex, basierend auf Rhodium, Palladium, Nickel, Platin, Kobalt oder Ruthenium, spezielle Verwendung in der homogenen Katalyse.

In einer ganz besonderen Ausführungsform finden mindestens eine der organischen Phosphorverbindungen, die in den erfindungsgemäßen Herstellungsbeispielen mit TP1, TP2, TP3, TP4 oder TP5 bezeichnet sind und/oder ein Metallkomplex, basierend auf Rhodium, Palladium, Nickel, Platin, Kobalt oder Ruthenium, Verwendung in einem Verfahren zur Hydroformylierung von olefinhaltigen Gemischen.

In den erfindungsgemäßen Herstellungsbeispielen werden die beanspruchten Metallkomplexe weiter offenbart und charakterisiert.

Auch ein Gegenstand der Erfindung ist ein Verfahren zur Hydrocyanierung von Pentennitril-haltigen Strömen unter Verwendung einer katalytisch wirksamen Zusammensetzung, dadurch gekennzeichnet, dass die katalytisch wirksame Zusammensetzung eine organische Phosphorverbindung, die in den erfindungsgemäßen Herstellungsbeispielen mit TP1, TP2, TP3, TP4 oder TP5 bezeichnet sind, enthält. Weitere Details der Offenbarung befinden sich in den erfindungsgemäßen Verfahrensbeispielen.

Eine besondere Ausführungsform des erfindungsgemäßen Verfahrens zur Hydrocyanierung ist dadurch gekennzeichnet, dass die Hydrocyanierung unter Ausschluss einer Isomerisierung zu verzweigten Nitrilen erfolgt. Weitere Details der Offenbarung befinden sich in den erfindungsgemäßen Verfahrensbeispielen.

Ein weiterer Gegenstand der Erfindung ist ein Verfahren zur Hydrocyanierung von Butadien-haltigen Strömen unter Verwendung einer katalytisch wirksamen Zusammensetzung, dadurch gekennzeichnet, dass die katalytisch wirksame Zusammensetzung eine organische Phosphorverbindung, die in den erfindungsgemäßen Herstellungsbeispielen mit TP2 bezeichnet wird, enthält.

Eine besondere Ausführungsform des Verfahren zur Hydrocyanierung von Butadien-haltigen Strömen ist dadurch gekennzeichnet, dass Butadien mit einer n-/iso-Selektivität von mehr als 99 % zu linearen Pentennitrilen hydrocyaniert wird. Weitere Details der Offenbarung befinden sich in den erfindungsgemäßen Verfahrensbeispielen.

Auch ein Gegenstand der Erfindung ist ein Verfahren zur Hydroformylierung von ungesättigten Kohlenwasserstoffgemischen unter Verwendung einer katalytisch wirksamen Zusammensetzung, enthaltend ein Übergangsmetall der Gruppen 8 bis 10, dadurch gekennzeichnet, dass die katalytisch wirksame Zusammensetzung eine organische Phosphorverbindung, die in den erfindungsgemäßen Herstellungsbeispielen mit TP1, TP2, TP3, TP4 oder TP5 bezeichnet sind, aufweist.

Eine besondere Ausführungsform des erfindungsgemäßen Verfahrens zur Hydroformylierung von ungesättigten Kohlenwasserstoffgemischen ist dadurch gekennzeichnet, dass die katalytisch wirksame Zusammensetzung eine organische Phosphorverbindung, die in den erfindungsgemäßen Herstellungsbeispielen mit TP1, TP2, TP3, TP4 oder TP5 bezeichnet sind, und Rhodium enthält. Weitere Details der Offenbarung befinden sich in den erfindungsgemäßen Verfahrensbeispielen.

Eine weitere Ausführungsform des erfindungsgemäßen Verfahrens zur Hydroformylierung von ungesättigten Kohlenwasserstoffgemischen ist dadurch gekennzeichnet, dass als ungesättigtes Kohlenwasserstoffgemisch ein Strom enthaltend Olefine mit mindestens 4 bis 20 C-Atomen eingesetzt wird.

In einer bevorzugten Ausführungsform werden als ungesättigtes Kohlenwasserstoffgemisch folgende Ströme eingesetzt:
Raffinat I in handelsüblicher Zusammensetzung;
Raffinat III in handelsüblicher Zusammensetzung, enthaltend einen Rest an C4-Alkanen, lineare C4-Alkene sowie Isobuten und C5-Alkane;
sogenanntes Rohbutan, enthaltend C4-Alkane, lineare C4-Alkene und C5-Alkane; sogenanntes Dibuten, enthaltend mindestens 98 Massen-% C8-Olefine, bezogen auf die Gesamtmenge an C8-Olefinen, ausgewählt aus der Gruppe der Dimethylhexene, der Methylheptene sowie n-Octene;
sogenannte Tributene, enthaltend ein Gemisch aus mindestens 98 Massen-% C11- und C12-Olefinen, bezogen auf die Gesamtmenge an C11- und C12-Olefinen.

Weitere Details der Offenbarung befinden sich in den erfindungsgemäßen Verfahrensbeispielen.

Ein weiterer Gegenstand er Erfindung ist ein Verfahren zur Hydroaminoalkylierung von ungesättigten Kohlenwasserstoffgemischen unter Verwendung einer katalytisch wirksamen Zusammensetzung, enthaltend ein Übergangsmetall der Gruppen 8 bis 10, dadurch gekennzeichnet, dass die katalytisch wirksame Zusammensetzung eine organischen Phosphorverbindung, die in den erfindungsgemäßen Herstellungsbeispielen mit TP6 bezeichnet wird, aufweist.

Eine besondere Ausführungsform des erfindungsgemäßen Verfahrens zur Hydroaminoalkylierung ist dadurch gekennzeichnet, dass die katalytisch wirksame Zusammensetzung eine organische Phosphorverbindung, die in den erfindungsgemäßen Herstellungsbeispielen mit TP6 bezeichnet wird, und Rhodium enthält.

Eine weitere Ausführungsform des erfindungsgemäßen Verfahrens zur Hydroaminoalkylierung ist dadurch gekennzeichnet, dass die Chemoselektivität bezüglich des Produktamins größer 90 % ist und die Bildung von Nebenprodukten kleiner 10% ist. Weitere Details der Offenbarung befinden sich in den erfindungsgemäßen Verfahrensbeispielen.

Auch ein Gegenstand der Erfindung ist ein Verfahren zur Hydrierung von ungesättigten Kohlenwasserstoffgemischen unter Verwendung einer katalytisch wirksamen Zusammensetzung, enthaltend ein Übergangsmetall der Gruppen 8 bis 10, dadurch gekennzeichnet, dass die katalytisch wirksame Zusammensetzung eine organische Phosphorverbindung, die in den erfindungsgemäßen Herstellungsbeispielen mit TP1 bezeichnet wird, aufweist. Weitere Details der Offenbarung befinden sich in den erfindungsgemäßen Verfahrensbeispielen.

Ein weiterer Gegenstand er Erfindung ist ein Verfahren zur Hydrosilylierung von Carbonylverbindungen unter Verwendung einer katalytisch wirksamen Zusammensetzung, enthaltend ein Übergangsmetall der Gruppen 8 bis 10, dadurch gekennzeichnet, dass die katalytisch wirksame Zusammensetzung eine organische Phosphorverbindung, die in den erfindungsgemäßen Herstellungsbeispielen mit TP3 bezeichnet wird, aufweist.

Weitere Details der Offenbarung befinden sich in den erfindungsgemäßen Verfahrensbeispielen.

### Beispiele

Die nachfolgenden Beispiele sollen die Erfindung näher erläutern

### Erfindungsgemäße Herstellungsbeispiele

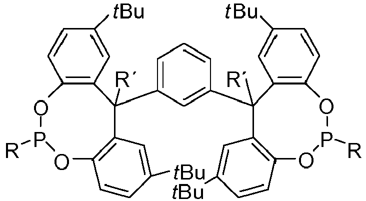

**Schema 1: Tetraphenol-Liganden TP1-7 und deren ³¹P{¹H} NMR Verschiebungen.**

| **Ligand** | **TP1** | **TP2** | **TP3** | **TP4** | **TP5** | **TP6** | **TP7** |
|---|---|---|---|---|---|---|---|
| **R** | 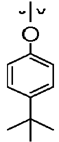 | 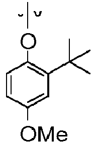 | 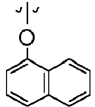 | 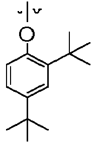 | 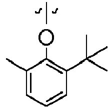 | 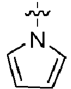 | 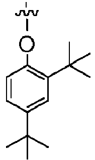 |

| **R'** | **H** | **H** | **H** | **H** | **H** | **H** | **CH₃** |
|---|---|---|---|---|---|---|---|
| **³¹P NMR d** | 122.1 | 122.6 | 119, 129 | 122.1 | 172.6 | 122.8 | 105.5 |

### Synthesebeispiele:

### Darstellung des Tetraphenolgrundgerüstes TP0:

### 4,4',4",4"'-Tetra-t-butyl-2,2',2", 2"'-(phenylenmethandiyl)tetraphenol

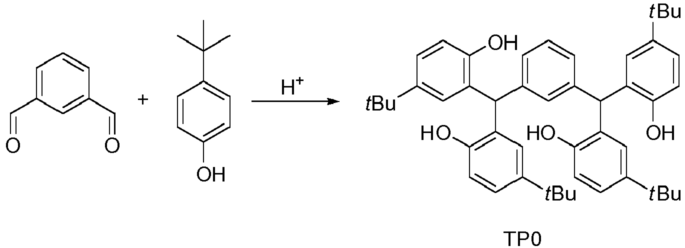

Das Tetraphenolgrundgerüst TP0 wurde nach einer modifizierten Literaturvorschrift [C. Gruettner, V. Boehmer, R. Assmus, S. Scherf, J. Chem. Soc. , Perkin Trans. 1 1995, 93-94.] dargestellt:
Eine Mischung aus 4-*tert*-Butylphenol (72 g, 0.48 mol) und Isophtaldicarboxaldehyd (8.1 g, 0.06 mol) wird unter Rühren erhitzt, bis eine homogene Schmelze entsteht (100-110 °C). Dann wird HCl (8 ml) zudosiert und weitere 6 Stunden gerührt. Das überschüssige Phenol wird mittels Wasserdampfdestillation entfernt und der Rückstand aus Aceton umkristallisiert. Ausbeute: 0.043 mol, 29.9 g, 71.5%. ¹H NMR (CDCl₃): δ 7.28-7.24 (m, 1H), 7.11-7.05 (m, 7H), 6.93 (d, J = 2.4 Hz, 4H), 6.71 (d, *J =* 8.4 Hz, 4H), 5.83 (s, 2H), 5.24 (bs, 4H, -OH), 1.13 (s, 36H). ¹³C NMR (CDCl₃): δ 150.93, 143.65, 141.99, 128.11, 127.28, 124.64, 115.74, 77.66, 77.02, 76.39, 45.14, 34.04, 31.40. Elementaranalyse für C₄₈H₅₈O₄*2(CH₃)CO: Berechnet (Gefunden): %C 79.57 (79.74), %H 8.66 (8.43).

### Darstellung des Tetraphenolgrundgerüstes TP'0

### 4,4',4",4'"-Tetra-t-butyl-2,2',2",2"'-(pheny/enemethylmethanediyl)tetrapheno/

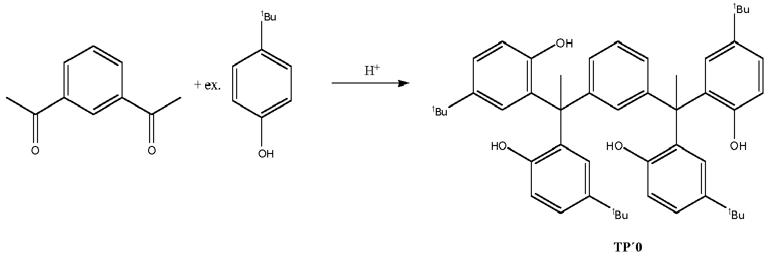

Eine Mischung aus 4-*tert*-Butylphenol (138.7 g, 0.924 mol) und 1,3-Diacetylbenzol (10.0 g, 0.0616 mol) wird unter Rühren erhitzt, bis eine homogene Schmelze entsteht (140°C). Danach wird Methansulfonsäure (5.3 g, 0.0553 mol, 3.59 ml) zugegeben und die Reaktionsmischung für 24 h erhitzt. Das überschüssige Phenol wird mittels Wasserdampfdestillation entfernt und der Rückstand aus Aceton umkristallisiert. Ausbeute Rohprodukt: 46.28 mmol, 33.6 g, 75.1 %. ¹H NMR (CDCl₃): δ 7.44 (bs, 1H), 7.17 (dd, J= 8.4, 2.4 Hz , 2H), 7.10-7.08 (m, 1 H), 6.99-6.96 (m, 8H), 6.94 (d, *J =* 2.4 Hz, 4H), 4.58 (bs, 4H,-OH), 1.91 (s, 6H), 1.16 (s, 36H).

### Darstellung des Tetraphenolgrundgerüstes TP"0

### 6,6',6",6"'-Tetra-o-methyl-2,2',2",2"'-(phenylenemethanediyl)tetraphenol

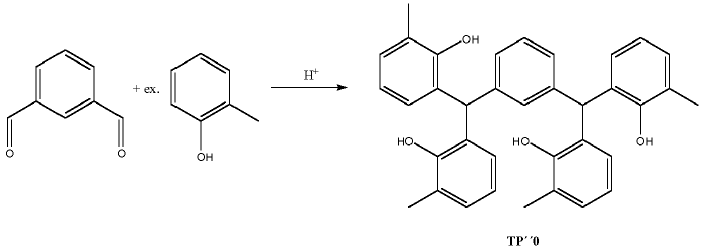

Eine Mischung aus *o*-Kresol (52 g, 0.48 mol) und Isophtaldicarboxaldehyd (8.1 g, 0.06 mol) wird unter Rühren erhitzt, bis eine homogene Schmelze entsteht (100-110 °C). Dann wird HCl (8 ml) zudosiert und weitere 6 Stunden gerührt. Danach wird das überschüssige Phenol mittels Wasserdampfdestillation entfernt und das Rohprodukt mittels NMR-Spektroskopie analysiert. Es wurden Reinheiten des Zielproduktes von 90-95 % detektiert. Ausbeute Rohprodukt (enthält noch *o*-Kresol): 34.3 g, 96 %. ¹H NMR (CDCl₃): δ 7.16-7.13 (m, 1 H), 7.00-6.88 (m, 4H), 6.84-6.74 (m, 8H), 6.70-6.69 (m, 1 H), 6.65-6.61 (m, 2H), 5.28 (s, 2H), 4.91 (bs, 4H, -OH), 2.16 (s, 12H).

### Darstellung des Bisphosphits TP1

Zu einer Lösung aus PCl₃ (0.6 mg, 6.5 mmol) in 35 ml THF werden das Tetraphenolgrundgerüst TP0 (2.1 g, 3 mmol) und Et₃N (5.4 ml, 36 mmol) bei -10°C zugetropft und die Lösung 30 min gerührt. Danach wird 4-*tert*-Butylphenol (6.05 mmol) gelöst in 10 ml THF bei -10°C zugetropft. Die Lösung wird eine Stunde bei Raumtemperatur gerührt, das Salz über einer Schicht von basischem Aluminiumoxid (4 cm) abfiltriert und das Filtrat zur Trockne eingeengt. Ausbeute: 0.96 mmol, 1.01 g, 32 %. ¹H NMR (CDCl₃): δ 7.38-7.35 (m, 2H), 7.33-7.30 (m, 2H), 7.24 (d, *J* = 2.9 Hz, 4H), 7.20-7.06 (m, 8H), 6.99 (dd, *J* = 8.5, 0.9, 4H), 6.85-6.77 (m, 4H), 5.77 (s, 2H), 1.08 (s, 54H). ¹³C NMR (CD₂Cl₂): 150.08, 149.96, 147.34, 146.99, 143.45, 134.50, 128.58, 128.14, 127.94, 126.48, 126.37, 126.24, 126.14, 125.47, 125.31, 123.40, 120.31, 120.22, 120.16, 53.24, 34.32, 34.25, 31.83, 31.14. ³¹P NMR (CD₂Cl₂): δ 122.08. Elementaranalyse für C₆₈H₈₀O₆P₂: Berechnet (Gefunden): %C 77.39 (77.33), %H 7.64 (7.45).

### Darstellung des Bisphosphits TP2

Vorschrift analog zu TP1, anstelle von 4-*tert*-Butylphenol wird 4-Hydroxy-3-*tert*-butylanisol zugegeben.
Ausbeute: 0.84 mmol, 937 mg, 28 %. ¹H NMR (CDCl₃): δ 7.47-7.43 (m, 2H), 7.33 (s, 4H), 7.11-6.96 (m, 12H), 6.48-6.43 (m, 4H), 5.72 (s, 2H), 3.29 (s, 6H), 1.36 (s, 18H), 1.09 (s, 36H). ¹³C NMR (CD₂Cl₂): 155.57, 147.77, 146.54, 146.43, 144.89, 144.60, 141.82, 141.78, 133.76, 128.87, 128.74, 128.06, 127.80, 127.02, 126.48, 125.29, 125.14, 122.93, 120.41, 120.11, 114.32, 109.99, 55.57, 45.57, 34.67, 34.16, 31.11, 29.1462. ³¹P NMR (CDCl₃): δ 122.61.

### Darstellung des Bisphosphits TP3

Vorschrift analog zu TP1, anstelle von 4-*tert*-Butylphenol wird 1-Naphthol zugegeben.
Ausbeute: 2.1 mmol, 2.2 g, 70.4 %. ¹H NMR (CDCl₃): δ 8.18 (d, *J* = 8Hz, 2H), 7.82 (d, *J =* 8Hz, 2H), 7.68-7.60 (m, 2H), 7.46-7.42 (m, 3H), 7.42-7.47 (m, 8H), 7.28 (s, 4H), 7.09-7.05 (m, 3H), 6.95-6.85 (m, 6H), 5.60 (s, 2H), 1.13 (s, 36H). ¹³C NMR (CD₂Cl₂): 147.71, 147.63, 146.44, 146.32, 134.87, 134.59, 128.72, 127.64, 127.52, 127.44, 127.35, 126.62, 126.51, 126.25, 126.08, 125.84, 125.73, 125.58, 125.26, 124.24, 123.82, 123.12, 122.54, 122.28, 115.10, 114.87, 95.04, 34.32, 31.43. ³¹P NMR (CDCl₃): δ 131.38. Elementaranalyse für C₆₈H₆₈O₆P₂: Berechnet (Gefunden): %C 78.29 (77.93), %H 6.57 (6.23).

### Darstellung des Bisphosphits TP4

Vorschrift analog zu TP1, anstelle von 4-*tert*-Butylphenol wird 2,4-Di-*tert*-butylphenol zugegeben.
Ausbeute: 2.4 mmol, 2.8 g, 80.7 %. ¹H NMR (CDCl₃): δ 7.30 (d, *J =* 2.5 Hz, 2H), 7.28-7.20 (m, 8H), 7.08-7.01 (m, 6H), 6.78-6.74 (m, 2H), 6.61 (dt, *J* = 4.9 Hz, 0.8 Hz, 4H), 5.83 (s, 2H), 1.42 (s, 18H), 1.29 (s, 54H). ¹³C NMR (CD₂Cl₂): 151.58, 149.08, 147.85, 147.49, 146.54, 146.08, 145.75, 138.95, 133.85, 128.79, 126.45, 125.34, 124.92, 124.53, 124.03, 123.50, 123.02, 119.89, 119.07, 118.84,118.70, 110.41, 45.76, 34.90, 34.82, 34.33, 34.22, 31.18, 29.95. ³¹P NMR (CD₂Cl₂): δ 122.12. Elementaranalyse für C₇₆H₉₆O₆P₂*CH₂Cl₂: Berechnet (Gefunden): %C 73.84 (74.32), %H 7.89 (8.19).

### Darstellung des Bisphosphits TP5

Vorschrift analog zu TP1, anstelle von 4-*tert*-Butylphenol wird 2-*tert*-Butyl-6-methylphenol zugegeben.
Ausbeute: 2.3 mmol, 2.5 g, 77.5 %. ¹H NMR (CDCl₃): δ 7.30 (d, *J =* 2.5 Hz, 2H), 7.26-7.20 (m, 6H), 7.13-7.03 (m, 8H), 7.01-6.90 (m, 4H), 6.77-6.65 (m, 2H), 6.22 (s, 2H), 2.52 (s, 6H), 1.49 (s, 54H). ¹³C NMR (CD₂Cl₂): 149.44, 149.35, 147.82, 147.53, 146.92, 146.88, 145.97, 145.80, 139.54, 139.30, 139.21, 138.97, 128.70, 127.58, 124.68, 124.57, 124.40, 123.94, 123.16, 120.48, 115.12, 114.88, 35.08, 32.53, 31.27, 30.00, 25.78. ³¹P NMR (CD₂Cl₂): δ 172.59. Elementaranalyse für C₇₀H₈₄O₆P₂*CH₂Cl₂: Berechnet (Gefunden): %C 72.99 (72.32), %H 7.42 (7.57).

### Darstellung des Bisphosphoramidits TP6

Vorschrift analog zu TP1, anstelle von 4-*tert*-Butylphenol wird Pyrrol zugegeben. ³¹P NMR (CD₂Cl₂): δ 122.77.

### Darstellung des Bisphosphits TP7

Das Tetraphenolgrundgerüst TP'0 (0.3 g, 0.41 mmol) und Et₃N (0.2 ml, 1.4 mmol) werden bei -10 °C zu einer Lösung aus PCl₃ (0.1 mg, 1.13 mmol) in 5 ml THF zugetropft und die Reaktionslösung 30 min gerührt. Danach wird 2,4-Di-*tert*-butylphenol (0.17 g, 0.825 mmol), gelöst in 1 ml THF, bei -10 °C zugetropft und anschließend 1 h bei Raumtemperatur gerührt. Das Salz wird über einer Schicht von basischem Aluminiumoxid (4 cm) abfiltriert und das Filtrat zur Trockne eingeengt. Ausbeute: 0.14 mmol, 0.165 g, 33.3 %. ¹H NMR (CDCl₃): δ 7.44 (bs, 1H), 7.17 (dd, J= 8.4, 2.4 Hz , 2H), 7.10-7.08 (m, 1H), 6.99-6.96 (m, 12H), 6.93 (d, *J* = 2.4 Hz, 6H), 1.91 (s, 6H), 1.42 (s, 9H), 1.29 (s, 9H), 1.16 (s, 54H). ³¹P NMR (CD₂Cl₂): δ 105.53.

### Darstellung des Tetraphosphits TP8

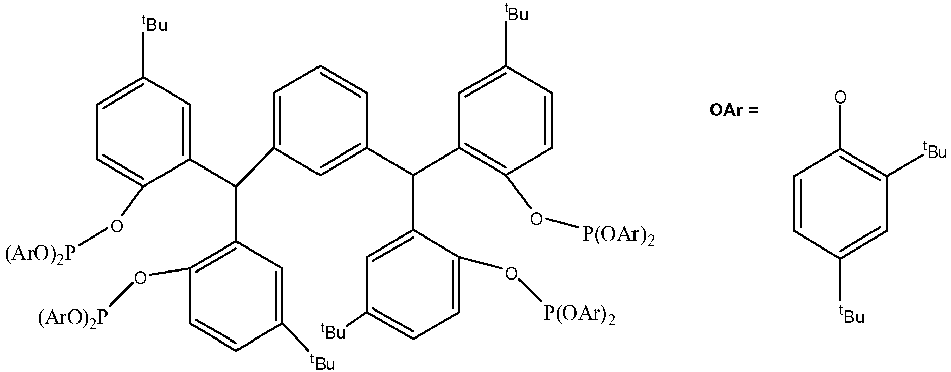

Zu einer Lösung aus PCl₃ (0.3 ml, 3.25 mmol) in 30 ml THF werden bei -10 °C nacheinander 2,4-Di-*tert*-butylphenol (1.355 g, 6.5 mmol) und Et₃N (2.4 ml, 16.0 mmol) tropfenweise zugegeben und für 30 min gerührt. Danach wir eine Lösung des Tetraphenolgrundgerüstes TP0 (0.57 g, 0.812 mmol) in 5 ml THF bei -10 °C zugetropft und 1 h bei Raumtemperatur gerührt. Das Salz wird über einer Schicht von basischem Aluminiumoxid (4 cm) abfiltriert und das Filtrat zur Trockne eingeengt. ³¹P NMR (THF): δ 128.0.

### Synthese von Platinkomplex (TP1)PtCl₂

Pt(cod)Cl₂ (35 mg, 94 µmol) und TP1 (121 mg, 113 µmol) wurden in 4 ml CH₂Cl₂/CH₃CN (3/2-Mischung) 1 h bei Raumtemperatur gerührt. Nach einer Woche bei -30 °C wurden für eine Röntgenkristallstrukturanalyse geeignet Kristalle erhalten. Ausbeute: 86.8 mg, 65.7 µmol, 62 %. ¹H NMR (CD₂Cl₂): δ 7.45 (dd, *J* = 8.7 Hz, 2.0 Hz, 4H), 7.40-7.00 (m, 16H), 6.79-6.81 (m, 4H), 5.56 (s, 2H) 1.41 (s, 18H), 1.32 (s, 36H). ¹³C NMR (CD₂Cl₂): δ 154.29, 153.15, 152.15 149.82, 143.32, 138.11, 133.67, 132.97, 132.62, 130.91, 130.68, 130.18, 125.78, 123.63, 61.36, 38.77, 35.46. ³¹P NMR (CD₂Cl₂/CH₃CN=3/2): δ 45.57 (*J*_{Pt-P}= 6091.46 Hz).

### Synthese von Nickelkomplexen (L)Ni(CO)₂ [C. J. Cobley and P. G. Pringle, Inorg. Chim. Acta 1997, 265, 107-115]

10 mg (0.036 mmol) Ni(cod)₂ und 1 Moläquivalent TP-Ligand (0.036 mmol) wurden in 2 mL Toluol gelöst. Die hellgelbe Lösung wurde 30 min mit CO durchströmt und wurde daraufhin farblos. Es wird bis zur Trockne im Vakuum eingeengt und der verbleibende Feststoff mittels ATR-IR spektroskopisch untersucht:

| Ligand | A₁ (cm⁻¹) | B₁(cm⁻¹) |
|---|---|---|
| TP1 | 2043 | 1995 |
| TP2 | 2040 | 1987 |
| TP3 | 2043 | 1991 |
| TP4 | 2041 | 1990 |
| TP5 | 2043 | 2002*^{a}* |

| | | |
|---|---|---|
| *^{a} mehrere Spezies vorhanden* | | |

### Synthese von (TP2)Ni(cod)

Eine Lösung von TP2 (22.0 mg, 0.018 mmol) in 1 mL Benzol-*d6* wurde zu Ni(cod)₂ (5.0 mg, 0.018 mmol) gegeben und in einem Schlenkgefäß 30 min gerührt. ¹H NMR (500 MHz, C₆D₆) δ (ppm): 8.75 (d, J = 8.5 Hz), 7.75 (s), 7.48 (d, J = 8.5 Hz), 7.23-7.19 (m), 7.12 (s), 7.07 (s), 7.03 (s), 6.99 (s), 6.44 (d, J = 8.5 Hz), 5.56 (s), 5.27 (s), 3.51 (s), 3.35 (s), 2.11 (s), 1.69 (s), 1.58-1.54 (m), 1.48 (s), 1.42-1.04 (m), 0.99 (s). ³¹P NMR (202 MHz, CDCl₃) δ (ppm): 124.6 (s).

### Synthese von (TP2)Ni(2M3BN)-ZnCl₂

Eine Lösung von TP2 (89.0 mg, 0.079 mmol) in 3 mL Toluol-d8 wurde zu Ni(cod)₂ (22.0 mg, 0.079 mmol) gegeben und in einem Schlenkgefäß 5 min gerührt. 2M3BN (10 µL, 1 eq.) wurde mittels einer Eppendorf-Pipette zugegeben sowie ZnCl₂ als Lewis-Säure (22.0 mg, 1 equiv.). Die Lösung wurde 30 min gerührt, eine Probe (800 µL) für die NMR-Analytik entnommen und die verbleibende Lösung im Vakuum zur Trockne eingeengt. Das rotorangefarbene Pulver wurde IR-spektroscopisch untersucht. IR (cm⁻¹) v□ : 3061 [C=(C-H)]; 2150 (CN). ¹H NMR (400 MHz, C₆D₆) δ (ppm): 8.26 (br s), 7.59-6.94 (m), 6.47 (d, J = 8.4 Hz), 6.21-6.10 (m), 5.08 (m), 4.78 (br s), 4.42 (br s), 4.16 (br s), 3.95 (br s), 3.83 (br s), 3.64 (br s), 3.44-3.40 (m), 3.37 (s), 3.36 (d, J = 2 Hz), 3.34-3.30 (m), 3.30 (d, J = 3 Hz), 3.28 (s), 2.92 (br s), 2.62 (br s), 1.59-0.82 (m), 0.64 (d, J = 6.8 Hz), 0.25 (s). ¹³C NMR (100.6 MHz, C₆D₆) δ (ppm): 156.46, 153.72, 148.49, 148.27, 146.70, 137.10, 129.15, 128.88, 126.55, 125.29, 124.12, 116.64, 115.94, 114.02, 110.54, 70.49, 69.44, 66.56, 55.71, 54.87, 35.54, 34.26, 33.94, 30.95, 29.95, 29.82, 29.31, 28.91, 27.98, 14.94, 1.01. ³¹P NMR (400 MHz, C₆D₆) δ (ppm): 124.6 (bs). Maldi-Tof:1114.39 (TP2), 1172.36 (TP2Ni), 1227.40 (TP2Ni(2M3BN)-CN).

### Erfindungsgemäße Verfahrensbeispiele:

### Hydrocyanierung

Nickel-katalysierte Hydrocyanierung von 3-Pentennitril (3PN): Zu Ni(cod)₂ (5.0 mg, 0.018 mmol) wird die Ligandlösung (0.018 mmol TP-Ligand in 2 mL Lösungsmittel) gegeben. Danach wird 3-Pentennitril (300 µL, 170 eq.) mittels einer Eppendorf-Pipette zugegeben, sowie 50 µL n-Decan als interner Standard und die Lewis-Säure (1 eq.). Die erhaltene Lösung wird in ein 15 ml Schlenkgefäß überführt. Dann wird Acetoncyanhydrin (400 µL, 250 eq.) mittels einer Eppendorf-Pipette zugegeben und das Schlenkgefäß bis 90 °C im Ölbad erwärmt. Die Lösung wird 4 h gerührt, dann auf 0 °C gekühlt und mit einem Argonstrom für 1 min gestrippt, um HCN-Spuren zu entfernen. Die Proben werden gaschromatographisch mit n-Decan als internem Standard bestimmt. Alle Reactions wurden doppelt durchgeführt, wobei eine Schwankungsbreite von Umsatz und Selektivität von ±2 % bzw. ±1 % festgestellt wurde.

**Tabelle 1. Hydrocyanierung von 3PN (3-Pentennitril) mit den Liganden TP1-TP5**

| Eintrag | Ligand | Umsatz^{a}% | 2PN^{b}% | 4PN^{b}% | Ausbeute DN^{c} % | ADN/MGD |
|---|---|---|---|---|---|---|
| 1 | TP1 | 24 | 10 | / | 14 | 83/17 |
| 2 | TP2 | 39 | 0 | 19 | 20 | 96/4 |
| 3 | TP3 | 15 | 1 | 7 | 7 | 75/25 |
| 4 | TP4 | 14 | 1 | 1 | 12 | 59/41 |
| 5 | TP5 | 15 | 1 | 8 | 6 | 68/32 |

| | | | | | | |
|---|---|---|---|---|---|---|
| *Bedingungen: 0.018 mmol Ni(cod)₂, Ni :L :Zn :S :ACH=1 :1 :1 :170 :Überschuss, Acetoncyanhydrin (ACH) als HCN-Quelle, T = 90°C, 2 mL Toluol, t = 4h.* *a) Gaschromatographische Bestimmung mit n-Decan als internem Standard. Die Umsätze basieren auf der Menge nicht-umgesetzten Substrates [mmol].* *b) Ausbeute von 2-Pentennitril bzw. 4-Pentennitril; c) Ausbeute Dinitrile: Adiponitril (ADN) + Methylglutarnitril (MGD).* | | | | | | |

**Tabelle 2. Hydrocyanierung von 3PN mit dem Liganden TP2**

| Eintrag | Lewis Säure | Umsatz^{a}% | Andere Nitrile^{b} (%) [2PN-4PN-2M2BN] | Ausbeute DN^{c} (%) [ADN/MGD] |
|---|---|---|---|---|
| 6 | ZnCl₂ (1eq) | 41 | [4-5-0] | 32 [88/12] |
| 7 | ZnCl₂ (2eq) | 40 | [4-2-0] | 34 [88/12] |
| 8 | ZnCl₂ (5eq) | 14 | [1-1-0] | 12 [85/15] |
| 9 | ZnCl₂ (3eq) | 53 | [3-11-0] | 38 [85/15] |
| 10^{d} | ZnCl₂ (3eq) | 29 | [3-9-0] | 20 [87/13] |
| 11 | AlCl₃ (1eq) | 1 | [0.2-0.4-0] | 0.1 [100/0] |
| 12^{e} | ZnCl₂ (3eq) | 43 | [3-17-1] | 22 [82/18] |

| | | | | |
|---|---|---|---|---|
| *Bedingungen: 0.018 mmol Ni(cod)₂, Ni :L :Zn :S :ACH=1 :1 :1 :170 : Überschuss, Acetoncyanhydrin (ACH) als HCN-Quelle, T = 90°C, 2 mL THF, t = 4h, TP2 als Ligand.* *a) Gaschromatographische Bestimmung mit n-Decan als internem Standard. Die Umsätze basieren auf der Menge nicht-umgesetzten Substrates [mmol].* *b) Ausbeute von 2-Pentennitril, 4-Pentennitril und 2-Methyl-2-Butennitril; c) Ausbeute Dinitrile: Adiponitril* + *Methylglutarnitril; d) T = 110* °*C; e) BIPPP als Benchmark-Ligand mit folgender Struktur:* | | | | |

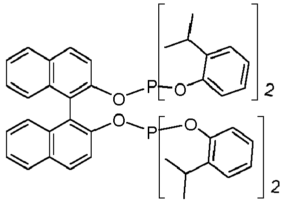

Binaphthyl-Phosphitligand (BIPPP) als Benchmark-Ligand

**Tabelle 3. Hydrocyanierung von 4PN in THF mit dem Liganden TP2**

| Eintrag | Umsatz^{a}% | 2PN^{b}% | 3PN^{b}% | Ausbeute DN^{c}% | ADN/MGD |
|---|---|---|---|---|---|
| 1 | 98.5 | 3.6 | 53 | 42 | 89/11 |

| | | | | | |
|---|---|---|---|---|---|
| *Bedingungen: 0.018 mmol Ni(cod)₂, Ni :L :Zn :S :ACH=1 :1 :1 :170 : Überschuss, Acetoncyanhydrin (ACH) als HCN-Quelle, T = 90°C, 2 mL THF, t = 4h, TP2 als Ligand.* *a) Gaschromatographische Bestimmung mit n-Decan als internem Standard. Die Umsätze basieren auf der Menge nicht-umgesetzten Substrates [mmol].* *b) Ausbeute von 2-Pentennitril bzw. 3-Pentennitril; c) Ausbeute Dinitrile: Adiponitril (ADN) + Methylglutarnitril (MGD).* | | | | | |

**Tabelle 4. Hydrocyanierung von Butadien in Dioxan mit verschiedenen Liganden**

| Eintrag | Ligand | Umsatz^{a}% | 3PN % | 2M3BN % | Andere Nitrile % |
|---|---|---|---|---|---|
| 1 | TP2 | 1.6 | 100 | / | / |
| 2 | Phosphit^{b} | 78.3 | 63.2 | 34.1 | 2.7 |
| 3 | Sixantphos | 80.2 | 53.1 | 41.5 | 5.4 |

| | | | | | |
|---|---|---|---|---|---|
| *Bedingungen: 0.018 mmol Ni(cod)₂, Ni :L :Zn :S :ACH=1 :1 :1 :125 : Überschuss, Acetoncyanhydrin (ACH) als HCN-Quelle, T = 90 °C, 2 mL Dioxan, t = 5 h.* *a) Gaschromatographische Bestimmung mit n-Decan als internem Standard. Die Umsätze basieren auf der Menge nicht-umgesetzten Substrates [mmol]; b) Phosphit* = [1,1']-Binaphthenyl-2,2'-bis[di-(2-isopropylphenyl)phosphit | | | | | |

*Allgemeine Vorschrift für Isomerisierungsexperimente:* Zu Ni(cod)₂ (5.0 mg, 0.018 mmol) wird die Ligandlösung (0.018 mmol TP-Ligand in 2 mL Lösungsmittel) gegeben und unter Inertgasatmosphäre 5 min gerührt. 2-Methyl-3-butennitril, 2M3BN, (200 µL, 100 eq.) wird mittels einer Eppendorf-Pipette zugegeben sowie 50 µL n-Decan als interner Standard und ZnCl₂ als Lewis-Säure (5.0 mg, 1 eq.). Das Schlenkgefäß wird auf 90 °C im Ölbad erwärmt und es werden regelmäßig Proben für die GC-Analytik entnommen. Die Selektivität wird als 3PN/(Σ nitriles) definiert.

### Hydroformylierung

Beispielhaft sind Reaktionen mit den Modellsubstraten 1-Octen, *trans*-2-Octen, *n-*Octengemisch, Dibuten, Isobuten und *cis*-2-Buten dargestellt. Die Rhodiumkonzentration betrug 40 und 200 ppm.

Beispielhaft sind im Folgenden die Ergebnisse der Hydroformylierung von 1-Octen und *trans-*2-Octen dargestellt. Diese Reaktionen wurden in einem AMTEC SPR16 Parallelreaktor durchgeführt. Rh(acac)(CO)₂ (3.7 mg, 14.4 µmol) und 4 Moläquivalente Ligand (57.6 µmol) wurden in 5 mL Toluol gelöst und in den Argon-gefüllten Reaktionsbehälter überführt. Dieser wurde auf 80 °C temperiert und mit 20 bar Synthesegas beaufschlagt. Nach 2 h Präformationszeit wurde die Substratmischung (18 mmol 1-Octen und 6 mmol n-Decan als interner Standard) zugegeben. Bei 80 °C und 20 bar Synthesegas wurde die Reaktionslösung 24 h gerührt.

**Tabelle 5. Hydroformylierung von 1-Octen**

| **Ligand** | **Umsatz (%)** | **linear/verzweigt** | **Aldol-Produkt (%)** | **TOF^{a}** |
|---|---|---|---|---|
| TP1 | 98 | 4.6 | 0.1 | 800 |
| TP2 | 99 | 2.7 | 0.1 | 1400 |
| TP3 | 99 | 12.3 | 0.3 | 460 |
| TP4 | 99 | 7.4 | 0.5 | 300 |
| TP5 | 99 | 2.7 | 0.1 | 55 |

| | | | | |
|---|---|---|---|---|
| *Bedingungen: Rh:L:S = 1:4:1250, 80 °C, 20 bar CO*/*H₂ (1:1), Toluol, [Rh] = 1.92 mM, Rh-Precursor = Rh(acac)(CO)₂, Vₜₒₜ = 8 mL, t = 24 h; a) TOF-Bestimmung bei 20% Umsatz.* | | | | |

**Tabelle 6. Hydroformylierung von trans-2-Octen**

| **Ligand** | **Zeit (h)** | **Druck (bar)** | **Temperatur (°C)** | **Umsatz (%)** | **linear/verzweigt Verhältnis** | **TOF*^{a}*** |
|---|---|---|---|---|---|---|
| **TP2** | 18 | 20 | 80 | 17 | 0.18 | 11 |
| **TP3** | 18 | 20 | 80 | 11 | 0.67 | 8 |
| **TP2** | 25 | 10 | 140 | 67 | 1.27 | 56 |
| **TP3** | 25 | 10 | 140 | 39 | 1.92 | 68 |
| **TP3*^{b}*** | 35 | 10 | 140 | 45 | 1.55 | 10 |

| | | | | | | |
|---|---|---|---|---|---|---|
| *Bedingungen: Rh:L:S = 1:4:1250, Toluol, [Rh] = 1.92 mM, Rh-Precursor = Rh(acac)(CO)₂; a) TOF-Bestimmung bei 20* % *Umsatz; b) Rh:L = 1:20* | | | | | | |

Aus der Dimerisierung von C4-Olefinen erhaltenes Dibuten (C8-Olefingemisch, linear und verzweigte Isomere) wurde lösungsmittelfrei mit dem Liganden TP1 hydroformyliert:

**Tabelle 7. Hydroformylierung von Dibuten (C8-Olefingemisch, linear und verzweigte Isomere)**

| **Ligand** | **Olefin** | **Druck (bar)** | **Temperatur (°C)** | **Umsatz (%)** | **Selektivität^{a} (%)** |
|---|---|---|---|---|---|
| **TP1** | Dibuten | 60 | 115 | 49 | 99 |
| **TP1** | Dibuten | 260^{b} | 135 | 84 | 99+ |

| | | | | | |
|---|---|---|---|---|---|
| *Bedingungen: Rh:L = 1:4; 50 bar CO*/*H₂ (1:1), 6 h, [Rh] = 40 ppm, lösungsmittelfrei, 1 kg Di-n-Buten; a) Chemoselektivität = Aldehyde*/*Umsatz; b) [Rh] = 20 ppm.* | | | | | |

Die folgenden Beispiele zur Hydroformylierung von C4-Olefinen (Tabelle 8) und C8-Olefinen (Tabelle 9) wurden in 100 ml Parr-Autoklaven mit Druckkonstanthaltung, Gasflussmessung und Flügelrührer durchgeführt. Der Autoklav wurde unter Argonatmosphäre mit allen unten genannten Verbindungen, jedoch noch nicht mit dem zu hydroformylierenden Olefingemisch befüllt. Nach Austausch der Argonatmosphäre durch Spülen mit Synthesegas (CO/H₂ 1:1) wurde das Reaktionsgemisch unter Rühren (1000 U/min) und unter Synthesegasdruck auf die jeweils genannte Temperatur aufgeheizt und danach der genaue Solldruck von 20 bar eingestellt. Anschließend wurde das zu hydroformylierende Olefingemisch zugegeben. Der Synthesegasdruck wurde während der gesamten Reaktionsdauer über einen Druckregler konstant gehalten. Die Reaktionsdauer der Hydroformylierungsversuche betrug jeweils 720 min, wobei zwischenzeitlich Proben aus dem Autoklaven für die GC-Analyse entnommen wurden. Anschließend wurde das Reaktionsgemisch auf Raumtemperatur abgekühlt, der Autoklav entspannt und mit Argon gespült.

**Tabelle 8. Hydroformylierung von C4-Olefinen**

| **Ligand** | **Olefin** | **Druck (bar)** | **Temperatur (°C)** | **Umsatz (%)** | ***n*-Selektivität^{a} (%)** | **k (min⁻¹)** |
|---|---|---|---|---|---|---|
| **TP1** | *cis-2-*Buten | 20 | 120 | 99 | 60 | 0.0089 |
| **TP2** | *cis-2-*Buten | 20 | 120 | 97 | 38 | 0.0047 |
| **TP4** | *cis-2-*Buten | 20 | 120 | 98 | 40 | 0.0063 |
| **TP1** | Isobuten | 20 | 120 | 91 | - | 0.0043 |
| **TP2** | Isobuten | 20 | 120 | 54 | - | 0.0011 |
| **TP4** | Isobuten | 20 | 120 | 60 | - | 0.0014 |

| | | | | | | |
|---|---|---|---|---|---|---|
| *Bedingungen: Rh:L = 1:4; 6 g C4-Olefin, 120 °C, 20 bar CO*/*H₂ (1:1), t = 720 min, Toluol, [Rh] = 40 ppm, Rh-Precursor = Rh(acac)(CO)₂; a) n-Selektivität = Pentanal*/*Summe Aldehyde, zum Zeitpunkt t = 720 min* | | | | | | |

**Tabelle 9. Hydroformylierung von 1-Octen und n-Octengemisch**

| **Ligand** | **Olefin** | **Druck (bar)** | **Temperatur (°C)** | **Umsatz (%)** | ***n*-Selektivität^{a} (%)** | **K^{b} (min⁻¹)** |
|---|---|---|---|---|---|---|
| **TP1** | 1-Octen | 20 | 100 | 99 | 78 | 0.0009 |
| **TP2** | 1-Octen | 20 | 100 | 99+ | 71 | 0.0242 |
| **TP3** | 1-Octen | 20 | 100 | 99 | 95 | 0.0056 |
| **TP4** | 1-Octen | 20 | 100 | 99+ | 73 | 0.0035 |
| **TP5** | 1-Octen | 20 | 100 | 99 | 95 | 0.0063 |
| **TP1** | n-Octene | 20 | 100 | 59 | 47 | 0.0019 |
| **TP2** | n-Octene | 20 | 120 | 58 | 28 | 0.0012 |
| **TP3** | n-Octene | 20 | 120 | 17 | 70 | 0.0004 |
| **TP4** | n-Octene | 20 | 120 | 44 | 31 | 0,0008 |
| **TP5** | n-Octene | 20 | 120 | 16 | 64 | 0.0004 |

| | | | | | | |
|---|---|---|---|---|---|---|
| *Bedingungen: Rh:L = 1:4; 10 g C8-Olefin, 20 bar CO*/*H₂ (1:1), t = 720 min, Toluol, [Rh] = 40 ppm, Rh-Precursor = Rh(acac)(CO)₂; a) n-Selektivität = Nonanal*/*Summe Aldehyde, zum Zeitpunkt t = 720 min; b) k (min⁻¹) nach Isomerisierung, nicht die Anfangsgeschwindigkeit.* | | | | | | |

### Hydroaminomethylierung

### Hydroaminomethylierung von 1-Octen mit TP6

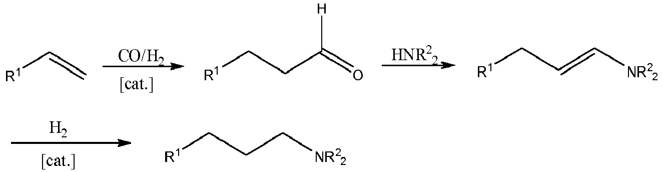

Für diese Kaskadenreaktion, bestehend aus Hydroformylierung und reduktiver Aminierung, wurde [Rh(cod)₂]BF₄ als Precursor eingesetzt. Die Reaktion wurde in einem Toluol/Methanol-Gemisch bei 110 °C und 36 bar CO/H₂ (1:2) und einer Rührergeschwindigkeit von 800 rpm durchgeführt. Nach 2 h wurde Vollumsatz erreicht, die Aktivität ist außergewöhnlich hoch! Diese Reaktion verläuft schnell und sehr chemoselektiv.

**Tabelle 10. Hydroaminomethylierung von 1-Octen und Piperidin mit Ligand TP6**

| Eintrag | t (min) | Umsatz (%) | Alken-Isomere (%) | Linear/ Verzweigt^{a} | Sel. Amin (%) |
|---|---|---|---|---|---|
| 1 | 26 | 92 | 2 | 10.4 | 48 |
| 2 | 51 | 98 | 1 | 8.5 | 69 |
| 3 | 96 | 99+ | - | 2.2 | 93 |
| 4 | 124 | 99+ | - | 2.0 | 95 |

| | | | | | |
|---|---|---|---|---|---|
| *a) bezogen auf die erhaltenen Amine.* | | | | | |

### Hydrierung

### Hydrierung von Itaconsäuredimethylester mit TP1

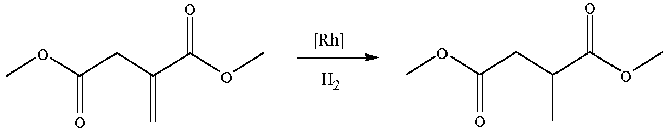

[Rh(cod)₂]BF₄ (4 mg, 9.85 µmol) wurde in 2 mL CH₂Cl₂ gelöst und zu TP1 (10 µmol) gegeben. Die gelbe Katalysatorlösung wurde 30 min bei Raumtemperatur gerührt, bevor sie zu einer Lösung aus Itaconsäuredimethylester (300 mg, 2 mmol) in 3 mL CH₂Cl₂ dosiert wurde. Diese Lösung wurde bei Raumtemperatur unter 1 bar H₂-Atmosphäre für 24 h gerührt. Der Substratumsatz beträgt 100 % (GC).

### Hydrosylilierung

### Hydrosylilierung von Acetophenon mit TP3

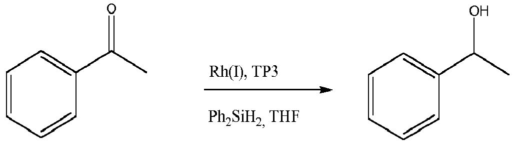

In einem 50 mL Schlenk-Gefäß werden zu einer Lösung aus [Rh(nbd)₂]BF₄ (11.5 mg, 4.75 µmol) und TP3 (150 mg, 14.25 µmol) in 3 ml THF 0.36 mL Acetophenon und 0.58 mL Diphenylsilan über eine Spritze zugetropft und die erhaltene Lösung unter Argon für 18 h bei Raumtemperatur gerührt. Danach wurden 6 ml HCl (10 % in H₂O) zugegeben und die Lösung zweimal mit jeweils 6 ml Diethylether extrahiert. Der Umsatz betrug 46 % (GC).

## Patentansprüche

1. Verfahren zur Hydrocyanierung von Pentennitril-haltigen Strömen unter Verwendung einer katalytisch wirksamen Zusammensetzung, enthaltend ein Übergangsmetall der Gruppen 8 bis 10, **dadurch gekennzeichnet, dass** die katalytisch wirksame Zusammensetzung eine organische Phosphorverbindung der Formel 2 aufweist, 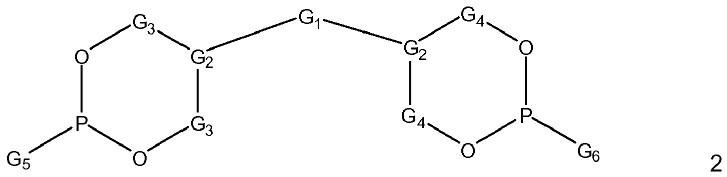 worin:
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist;
G3 und G4 gleich oder verschieden sind und eine mindestens einfach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden sind;
G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Acyl, N-Alkyl, N-Aryl, N-Heteroaryl, N-Cycloalkyl, N-Silyl.

2. Verfahren nach Anspruch 1, **dadurch gekennzeichnet, dass** die katalytisch wirksame Zusammensetzung eine organische Phosphorverbindung der Formel 2 aufweist, 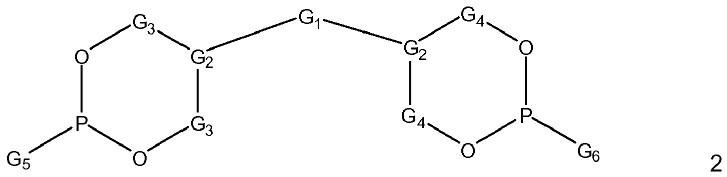 worin:
G1 ein mindestens disubstituierter 1,2-, 1,3- oder 1,4- Phenylrest ist;
G2 ein C1-Alkylrest ist, der tertiär oder quartär substituiert ist;
G3 und G4 gleich sind und einen mindestens einfach substituierten aromatischen Rest darstellen, ausgewählt aus der Gruppe der Aromaten, der Heteroaromaten, der kondensierten Aromatensysteme oder der kondensierten heteroaromatischen Systeme;
G5 und G6 gleich sind, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Alkyl, N-Aryl, N-Heteroaryl, N-Cycloalkyl, N-Silyl.

3. Verfahren nach den Ansprüchen 1 und 2, **dadurch gekennzeichnet, dass** die katalytisch wirksame Zusammensetzung eine organische Phosphorverbindung der Formel 2 aufweist, 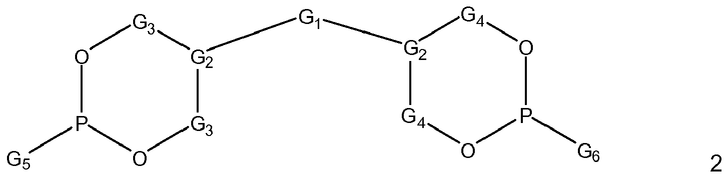 worin:
G1 ein 1,3-disubstituierter Phenylrest ist;
G2 ein C1-Alkylrest ist, der mit Wasserstoff oder Methyl substituiert ist;
G3 und G4 gleich sind, ausgewählt aus der Gruppe tert.-Butylphenoxy, Methoxy-tert.-Butylphenoxy, oder di-tert.-Butylphenoxy;
G5 und G6 gleich sind, ausgewählt aus der Gruppe tert.-Butylphenoxy, Methoxy-tert.-Butylphenoxy, Naphthoxy, di-tert.-Butylphenoxy, Methyl-tert.-Butylphenoxy oder Pyrrol; Ni als Übergangsmetall aufweist;
und zusätzlich eine Lewis-Säure, ausgewählt aus Zn²⁺, Al³⁺ enthält.

4. Verfahren nach mindestens einem der vorhergehenden Ansprüche, **dadurch gekennzeichnet, dass** die Hydrocyanierung unter Ausschluss einer Isomerisierung zu verzweigten Nitrilen erfolgt.

5. Verfahren zur Hydrocyanierung von Butadien-haltigen Strömen unter Verwendung einer katalytisch wirksamen Zusammensetzung, enthaltend ein Übergangsmetall der Gruppen 8 bis 10, **dadurch gekennzeichnet, dass** die katalytisch wirksame Zusammensetzung eine organische Phosphorverbindung der Formel 2 aufweist, 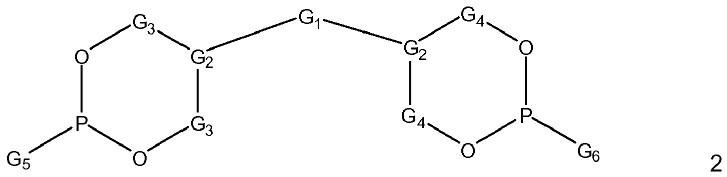 worin:
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme mit beliebiger weiterer Substitution, die jeweils monovalent mit G2 verbunden ist;
G2 einen Alkylrest darstellt und jeweils monovalent mit G1, G3 und/oder G4 verbunden ist;
G3 und G4 gleich oder verschieden sind und eine mindestens einfach substituierte cyclische Struktur darstellen, ausgewählt aus der Gruppe der Aromaten, der Heteroaromaten, der kondensierten Aromatensysteme oder der kondensierten heteroaromatischen Systeme mit beliebiger weiterer Substitution, welche jeweils monovalent mit G2 und O verbunden sind;
G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Acyl, N-Alkyl, N-Aryl, N-Heteroaryl, N-Cycloalkyl, N-Silyl.

6. Verfahren nach Anspruch 5, **dadurch gekennzeichnet, dass** die katalytisch wirksame
Zusammensetzung eine organische Phosphorverbindung der Formel 2 aufweist, 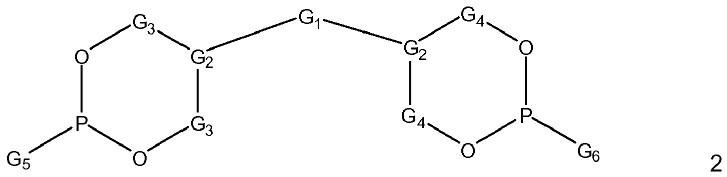 worin:
G1 ein mindestens disubstituierter 1,2-, 1,3- oder 1,4- Phenylrest ist;
G2 ein C1-Alkylrest ist, der tertiär oder quartär substituiert ist;
G3 und G4 gleich sind und einen mindestens einfach substituierten aromatischen Rest darstellen, ausgewählt aus der Gruppe der Aromaten, der Heteroaromaten, der kondensierten Aromatensysteme oder der kondensierten heteroaromatischen Systeme;
G5 und G6 gleich sind, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Alkyl, N-Aryl, N-Heteroaryl, N-Cycloalkyl, N-Silyl.

7. Verfahren nach den Ansprüchen 5 und 6, **dadurch gekennzeichnet, dass** die katalytisch wirksame Zusammensetzung eine organische Phosphorverbindung der Formel 2 aufweist, 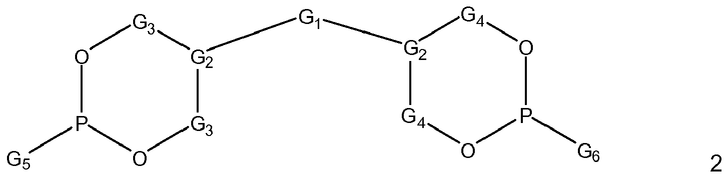 worin:
G1 ein 1,3-disubstituierter Phenylrest ist;
G2 ein C1-Alkylrest ist, der mit Wasserstoff oder Methyl substituiert ist;
G3 und G4 gleich sind, ausgewählt aus der Gruppe tert.-Butylphenoxy, Methoxy-tert.-Butylphenoxy, oder di-tert.-Butylphenoxy;
G5 und G6 gleich sind, ausgewählt aus der Gruppe tert.-Butylphenoxy, Methoxy-tert.-Butylphenoxy, Naphthoxy, di-tert.-Butylphenoxy, Methyl-tert.-Butylphenoxy oder Pyrrol; Ni als Übergangsmetall aufweist;
und zusätzlich eine Lewis-Säure, ausgewählt aus Zn²⁺, Al³⁺ enthält..

8. Verfahren nach mindestens einem der vorhergehenden Ansprüche, **dadurch gekennzeichnet, dass** Butadien mit einer n/iso-.Selektivität von mehr als 99 % zu linearen Pentennitrilen hydrocyaniert wird.

## Geänderte Patentansprüche

### Geänderte Patentansprüche gemäss Regel 137(2) EPÜ.

1. Verfahren zur Hydrocyanierung von Pentennitril-haltigen Strömen unter Verwendung einer katalytisch wirksamen Zusammensetzung, enthaltend ein Übergangsmetall, **dadurch gekennzeichnet, dass** die katalytisch wirksame Zusammensetzung eine organische Phosphorverbindung der Formel 2 aufweist, 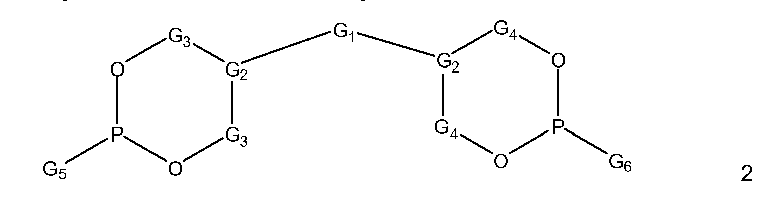 worin:
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme, die jeweils monovalent mit G2 verbunden ist;
G2 einen Alkylrest darstellt und jeweils monovalent mit G1, G3 und/oder G4 verbunden ist;
G3 und G4 gleich oder verschieden sind und eine mindestens einfach substituierte cyclische Struktur darstellen, ausgewählt aus der Gruppe der Aromaten, der Heteroaromaten, der kondensierten Aromatensysteme oder der kondensierten heteroaromatischen Systeme, welche jeweils monovalent mit G2 und O verbunden sind;
G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Acyl, N-Alkyl, N-Aryl, N-Heteroaryl, N-Cycloalkyl, N-Silyl.

2. Verfahren nach Anspruch 1, **dadurch gekennzeichnet, dass** die katalytisch wirksame Zusammensetzung eine organische Phosphorverbindung der Formel 2 aufweist, 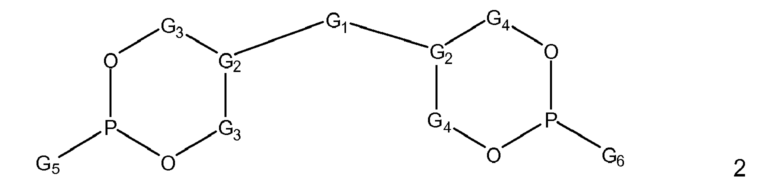 worin:
G1 ein mindestens disubstituierter 1,2-, 1,3- oder 1,4- Phenylrest ist;
G2 ein C1-Alkylrest ist, der tertiär oder quartär substituiert ist;
G3 und G4 gleich sind und einen mindestens einfach substituierten aromatischen Rest darstellen, ausgewählt aus der Gruppe der Aromaten, der Heteroaromaten, der kondensierten Aromatensysteme oder der kondensierten heteroaromatischen Systeme;
G5 und G6 gleich sind, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Alkyl, N-Aryl, N-Heteroaryl, N-Cycloalkyl, N-Silyl.

3. Verfahren nach den Ansprüchen 1 und 2, **dadurch gekennzeichnet, dass** die katalytisch wirksame Zusammensetzung eine organische Phosphorverbindung der Formel 2 aufweist, 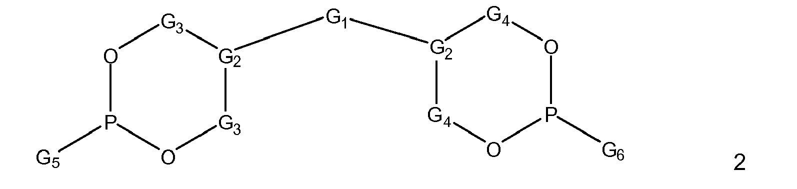 worin:
G1 ein 1,3-disubstituierter Phenylrest ist;
G2 ein C1-Alkylrest ist, der mit Wasserstoff oder Methyl substituiert ist;
G3 und G4 gleich sind, ausgewählt aus der Gruppe tert.-Butylphenoxy, Methoxy-tert.-Butylphenoxy, oder di-tert.-Butylphenoxy;
G5 und G6 gleich sind, ausgewählt aus der Gruppe tert.-Butylphenoxy, Methoxy-tert.-Butylphenoxy, Naphthoxy, di-tert.-Butylphenoxy, Methyl-tert.-Butylphenoxy oder Pyrrol;
Ni als Übergangsmetall aufweist;
und zusätzlich eine Lewis-Säure, ausgewählt aus Zn²⁺, Al³⁺ enthält.

4. Verfahren nach mindestens einem der vorhergehenden Ansprüche, **dadurch gekennzeichnet, dass** die Hydrocyanierung unter Ausschluss einer Isomerisierung zu verzweigten Nitrilen erfolgt.

5. Verfahren zur Hydrocyanierung von Butadien-haltigen Strömen unter Verwendung einer katalytisch wirksamen Zusammensetzung, enthaltend ein Übergangsmetall, **dadurch gekennzeichnet, dass** die katalytisch wirksame Zusammensetzung eine organische Phosphorverbindung der Formel 2 aufweist, 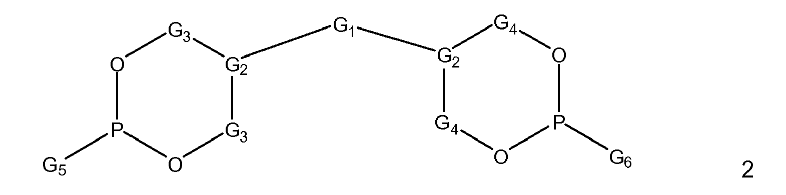 worin:
G1 eine mindestens zweifach substituierte cyclische Struktur darstellt, welche ausgewählt ist aus der Gruppe der Aromaten, der Heteroaromaten, der kondensierten Aromatensysteme oder der kondensierten heteroaromatischen Systeme, die jeweils monovalent mit G2 verbunden ist;
G2 einen Alkylrest darstellt und jeweils monovalent mit G1, G3 und/oder G4 verbunden ist;
G3 und G4 gleich oder verschieden sind und eine mindestens einfach substituierte cyclische Struktur darstellen, ausgewählt aus der Gruppe der Aromaten, der Heteroaromaten, der kondensierten Aromatensysteme oder der kondensierten heteroaromatischen Systeme, welche jeweils monovalent mit G2 und O verbunden sind;
G5 und G6 jeweils gleiche oder verschiedene monovalent mit P verbundene Einheiten darstellen, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Acyl, N-Alkyl, N-Aryl, N-Heteroaryl, N-Cycloalkyl, N-Silyl.

6. Verfahren nach Anspruch 5, **dadurch gekennzeichnet, dass** die katalytisch wirksame
Zusammensetzung eine organische Phosphorverbindung der Formel 2 aufweist, 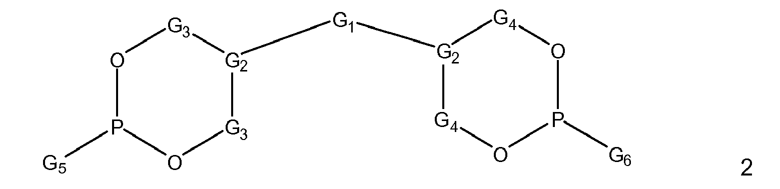 worin:
G1 ein mindestens disubstituierter 1,2-, 1,3- oder 1,4- Phenylrest ist;
G2 ein C1-Alkylrest ist, der tertiär oder quartär substituiert ist;
G3 und G4 gleich sind und einen mindestens einfach substituierten aromatischen Rest darstellen, ausgewählt aus der Gruppe der Aromaten, der Heteroaromaten, der kondensierten Aromatensysteme oder der kondensierten heteroaromatischen Systeme;
G5 und G6 gleich sind, ausgewählt aus der Gruppe O-Alkyl, O-Aryl, O-Acyl, O-Heteroaryl, O-Cycloalkyl, O-Silyl, Acyl, Alkyl, Aryl, Heteroaryl, Cycloalkyl, Perfluoralky, N-Alkyl, N-Aryl, N-Heteroaryl, N-Cycloalkyl, N-Silyl.

7. Verfahren nach den Ansprüchen 5 und 6, **dadurch gekennzeichnet, dass** die katalytisch wirksame Zusammensetzung eine organische Phosphorverbindung der Formel 2 aufweist, 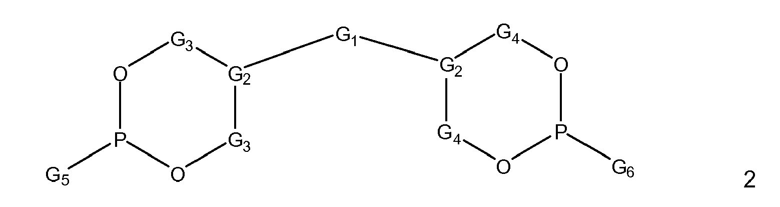 worin:
G1 ein 1,3-disubstituierter Phenylrest ist;
G2 ein C1-Alkylrest ist, der mit Wasserstoff oder Methyl substituiert ist;
G3 und G4 gleich sind, ausgewählt aus der Gruppe tert.-Butylphenoxy, Methoxy-tert.-Butylphenoxy, oder di-tert.-Butylphenoxy;
G5 und G6 gleich sind, ausgewählt aus der Gruppe tert.-Butylphenoxy, Methoxy-tert.-Butylphenoxy, Naphthoxy, di-tert.-Butylphenoxy, Methyl-tert.-Butylphenoxy oder Pyrrol;
Ni als Übergangsmetall aufweist;
und zusätzlich eine Lewis-Säure, ausgewählt aus Zn²⁺, Al³⁺ enthält..

8. Verfahren nach mindestens einem der vorhergehenden Ansprüche, **dadurch gekennzeichnet, dass** Butadien mit einer n/iso-.Selektivität von mehr als 99 % zu linearen Pentennitrilen hydrocyaniert wird.
